# EUROPÄISCHE PATENTSCHRIFT

(11) **EP 2 416 752 B1**
(45) Veröffentlichungstag und Bekanntmachung des Hinweises auf die Patenterteilung: **18.09.2013**
(21) Anmeldenummer: 10710576.9
(22) Anmeldetag: 22.03.2010
(51) Int. Cl.: A61K 8/11, A61K 8/26, A61K 8/29, A61K 8/34, A61K 8/73, A61Q 1/12, A61Q 5/00, A61Q 5/06, A61K 8/02, A61K 8/70

(54) **PULVERFÖRMIGE ZUSAMMENSETZUNG ZUR FORM- UND GLANZGEBUNG KERATINISCHER FASERN**
POWDERY COMPOSITION FOR SHAPING KERATIN FIBRES AND GIVING THEM SHINE
COMPOSITION PULVÉRULENTE POUR METTRE EN FORME DES FIBRES KÉRATINIQUES ET LEUR DONNER DE L'ÉCLAT

(30) Priorität: 07.04.2009 DE 102009002267
(43) Veröffentlichungstag der Anmeldung: 15.02.2012
(73) Patentinhaber: Henkel AG & Co. KGaA, 40589 Düsseldorf (DE)
(72) Erfinder: KNAPPE, Thorsten, 22869 Schenefeld (DE); RICHTERS, Bernd, 21129 Hamburg (DE); BAYERSDÖRFER, Rolf, 22589 Hamburg (DE); HENTRICH, Dirk, 22457 Hamburg (DE)
(86) Internationale Anmeldenummer: PCT/EP2010/053700
(87) Internationale Veröffentlichungsnummer: WO 2010/115700

(56) Entgegenhaltungen:
- US-A1- 2007 218 024
- US-A1- 2008 095 724

## Beschreibung

Die vorliegende Erfindung betrifft pulverförmige Zusammensetzungen zur temporären Verformung des menschlichen Haupthaars, enthaltend 50 bis 90 Gew.-% Wasser, 0,5 Gew.-% bis 10 Gew.-% mindestens einer weiteren Flüssigkeit, die einen Siedepunkt von mehr als 150°C bei einem Druck von 1013 mbar aufweist, mindestens eine Stärke, die mit Carboxy-(C₁ bis C₄)-alkylgruppen substituiert ist und Metalloxidpartikel, die an der Oberfläche mit fluorhaltigen, organischen Gruppen beschichtet wurden. Ein Verfahren zur temporären Verformung mit dieser Zusammensetzung sowie die Verwendung dieser Zusammensetzung zur Glanzgebung bzw. zur temporären Formgebung des menschlichen Haupthaars sind ebenso umfasst.

Stylingmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Bekannte Formen temporärer Stylingmittel lassen sich oftmals nicht mit zufriedenstellender Genauigkeit dosieren. So lassen sich etwa Haargele, Haarcremes und Haarwachse nur noch schwierig verteilen, sobald sie einmal auf das Haar aufgebracht sind. Sobald der Kamm oder die Hände, auf die das Stylingmittel aufgebracht wurde, mit den ersten Haarpartien in Kontakt kommen, werden vergleichsweise große Mengen an Stylingmittel an das Haar abgegeben. In Haarpartien, die erst später mit dem Kamm oder den Händen erreicht werden, wird hingegen vergleichsweise wenig Stylingmittel eingearbeitet. Dies hat zur Folge, dass der Anwender entweder von Anfang an eine große Menge Stylingmittel aufbringen muss, so dass auch die zuletzt erreichten Haarpartien ausreichend Stylingmittel erhalten, oder gezwungen ist, das Stylingmittel in mehreren Schritten aufzubringen, wobei jeweils andere Haarpartien behandelt werden. Haarsprays lassen sich gleichmäßiger auf das Haar verteilen. Da der Verwender aber keine Möglichkeit hat, die Gesamtmenge an aufgebrachtem Stylingmittel visuell zu erfassen, besteht die Gefahr, dass mehr Stylingmittel auf das Haar aufgebracht wird, als eigentlich erforderlich wäre.

Die bekannten Formen temporärer Stylingmittel erfordern zudem in der Regel eine große Menge an Hilfsstoffen, die nicht der eigentlichen Gestaltung der Frisur, sondern der Formulierung des jeweiligen Mittels dienen. So enthalten die Stylingmittel oftmals große Mengen organischer Lösungsmittel. Die Konfektionierung als Haarspray erfordert zudem weitere organische Verbindungen, die als Treibmittel Verwendung finden. Dies hat zum einen die Folge, dass die Umwelt mit flüchtigen organischen Verbindungen (VOC) belastet wird, zum anderen erhöht sich so das Produktvolumen und damit das Volumen der benötigten Verpackungen erheblich. Aufgabe der vorliegenden Erfindung war es daher, ein Haarbehandlungsmittel zur temporären Formgebung von menschlichem Haupthaar zur Verfügung zur stellen, das ein hervorragendes und haltbares Stylingergebnis ergibt und andererseits in möglichst kompakter Form vorliegt und genau und einfach dosiert werden kann.

Es wurde gefunden, dass dies auf einfache Weise durch ein Stylingmittel erreicht werden kann, das in Pulverform vorliegt.

Pulverförmige Kosmetika sind bekannt und werden etwa im Bereich der Hautbehandlung bereits seit langem eingesetzt. Typische Beispiele sind etwa Make-up Puder oder Lidschatten. Um die pulverförmige Konsistenz zu erzielen, ist der Einsatz eines pulverförmigen Trägermaterials erforderlich. Als geeignetes Trägermaterial kann etwa Siliciumdioxid verwendet werden. Besonders interessant ist hydrophobiertes Siliciumdioxid. Dieses kann beispielsweise ausgehend von pyrogenem Siliciumdioxid, das in verschiedenen Spezifikationen kommerziell erhältlich ist, erhalten werden. Auf der Oberfläche trägt unbehandeltes pyrogenes Siliciumdioxid Silanol- und Siloxangruppen. Dadurch hat es eine hohe Affinität zu Wasser, d.h. es ist hydrophil. Durch Umsetzung mit geeigneten organischen Siliciumverbindungen lassen sich Alkylsilylgruppen auf der Oberfläche des pyrogenen Siliciumdioxids chemisch binden. Es entstehen modifizierte Siliciumdioxidpulver, die nicht mehr von Wasser benetzt werden, d.h. die hydrophobe Eigenschaften aufweisen.

In Seifen, Öle, Fette, Wachse (SÖFW), 3 (2004), S. 4-13 wird der Einsatz von hydrophobem Siliciumdioxid in der Kosmetik zur Herstellung von so genanntem trockenen Wasser für die Haut beschrieben. Dabei werden die hydrophoben Eigenschaften des modifizierten Siliciumdioxids ausgenutzt, die bewirken, dass das Siliciumdioxid beim intensiven Mischen mit Wasser nicht einfach in diesem dispergiert wird. Die Wassertropfen werden vielmehr von den hydrophoben Feststoffpartikeln umhüllt und am erneuten Zusammenfließen gehindert. Auf diese Weise lassen sich pulverförmige Feststoffe mit einem Wassergehalt von bis zu über 95 % erhalten. Unter mechanischer Beanspruchung, beispielsweise beim Verreiben auf der Haut, wird das eingeschlossene Wasser wieder freigesetzt. Dieses trockene Wasser wird als Grundlage zur Herstellung von lagerstabilem, festem Wasserstoffperoxid und von verstreichbaren Zubereitungen mit sehr geringem Ölgehalt beschrieben.

Dieses Konzept liegt auch der in EP 1 235 554 B1 beschriebenen Herstellung kosmetischer oder pharmazeutischer, verflüssigbarer Pulverzusammensetzungen zu Grunde. Die Pulverzusammensetzungen umfassen hydrophob beschichtete Metalloxidteilchen, in die Wasser und ein wasserlösliches Polymer eingeschlossen sind, wobei die Zusammensetzungen weniger als 1 % Öl enthalten. Durch Zugabe des wasserlöslichen Polymers soll erreicht werden, dass sich das Pulver bei der Anwendung auf der Haut angenehm und nicht körnig anfühlt, ohne dass zu diesem Zweck dem Produkt eine Ölkomponente zugegeben werden müsste. Das Polymer wird zu diesem Zweck der Wasserphase in einer Menge von 0,01 bis 5 Gew.-% zugegeben, wobei ein Gehalt an lediglich 0,1 bis 1 Gew.-% bevorzugt ist. Die verflüssigbaren Pulverzusammensetzungen werden vor allem zur Herstellung dekorativer Kosmetika eingesetzt. Daneben sind auch der Einsatz in Deodorants oder Sonnenschutzmitteln, oder die Anwendung auf dem Haar als Basis von Haarbehandlungsmitteln, die Perlglanzmittel oder Pflegekomponenten enthalten, beschrieben. Eine Verwendung im Bereich der Stylingmittel ist nicht genannt.

In der Druckschrift WO 2007/051511 A1 wird die Verwendung pulverförmiger Zusammensetzungen zur temporären Haarumformung beschrieben. Diese pulverförmigen Zusammensetzungen enthalten hydrophobiertes Siliziumdioxid, 50 bis 90 Gew.-% Wasser und mindestens ein filmbildendes und/oder festigendes Polymer.

US 2008/095724 A1 (HASENZAHL STEFFEN [US] ET AL) 24. April 2008 (2008-04-24)
offenbart (Beispiel 1; Tabelle 1) eine Pulverförmige Zusammensetzung, enthaltend i) 88,9 Gew.-% Wasser,
ii) 5 Gew.-% Propylen Glykol
iii) 0,8% einer Stärke (Covagel)
iv) Metalloxidpartikel, die an der Oberfläche mit hydrophobisierten, organischen Gruppen beschichtet wurden (Aerosil, Tab. 1).

Als Nachteil der bekannten pulverförmigen Zusammensetzungen gilt die Tatsache, dass nach der Anwendung das Haar nahezu stumpf erscheint, d.h. keinen Glanz aufweist. Eine Aufgabe der vorliegenden Erfindung war es, neben der Erfüllung der zuvor genannten Parameter zusätzlich eine glanzbringende Zusammensetzung zur temporären Verformung des Haupthaars bereitzustellen.

Ein erster Gegenstand der vorliegenden Erfindung sind daher pulverförmige Zusammensetzungen zur temporären Verformung des menschlichen Haupthaars, enthaltend
i) 50 bis 90 Gew.-% Wasser,
ii) 0,5 Gew.-% bis 10 Gew.-% mindestens einer weiteren Flüssigkeit, die einen Siedepunkt von mehr als 150°C bei einem Druck von 1013 mbar aufweist,
iii) mindestens eine Stärke, die mit Carboxy-(C₁ bis C₄)-alkylgruppen substituiert ist,
iv) Metalloxidpartikel, die an der Oberfläche mit fluorhaltigen, organischen Gruppen beschichtet wurden.
v) mittels Triethoxy Caprylsilan beschichtete Metalloxidpartikel, die keine fluorierten organischen Gruppen tragen

Unter temporärer Verformung des menschlichen Haupthaars ist die temporäre Fixierung einer Frisur zu verstehen. Unter Haupthaar versteht der Fachmann solche Haare, die auf dem Kopf wachsen und nicht im Gesicht verwurzelt sind. Augenbrauen, Bart und Wimpern sind beispielsweise gemäß vorliegender Erfindung nicht von dem Begriff Haupthaar umfasst.

Partikel sind im Sinne der Erfindung als Korn vorliegende Teilchen (vgl. DIN 66160: 1992-09) von Festkörpern. Pulverförmig im Sinne der Erfindung sind Zusammensetzungen, deren Teilchen unter eigenem Gewicht frei rieselfähig sind (vgl. DIN EN ISO 6186: 1998-08).

Die erfindungsgemäßen pulverförmigen Zusammensetzungen können sehr einfach dosiert werden. Sie lassen sich zudem sehr gleichmäßig im Haar verteilen, da die bei Anwendungsbedingungen flüssigen Inhaltsstoffe des Pulvers und gegebenenfalls darin befindliche Wirkstoffe erst unter mechanischer Beanspruchung freigesetzt werden. Das Pulver kann also zunächst vorsichtig im Haar verteilt und erst anschließend stärker mechanisch belastet werden, beispielsweise durch gezieltes Einmassieren des Pulvers in das Haar. Dadurch wird die flüssige Phase erst direkt auf der gewünschten Haarpartie freigesetzt. So lässt sich sehr gezielt eine hervorragende Dosierung und Wirkung erreichen.

Die erfindungsgemäße pulverförmige Zusammensetzung umfasst bevorzugt Kern-Hülle-Teilchen, deren Hülle an der Oberfläche mit fluorhaltigen, organischen Gruppen beschichtete Metalloxidpartikel enthält und deren Kern eine flüssige, wässrige Phase umfasst, dadurch gekennzeichnet, dass die pulverförmige Zusammensetzung
i) 50 bis 90 Gew.-% Wasser,
ii) 0,5 Gew.-% bis 10 Gew.-% mindestens einer weiteren Flüssigkeit, die einen Siedepunkt von mehr als 150°C bei einem Druck von 1013 mbar aufweist,
iii) mindestens eine Stärke, die mit Carboxy-(C₁ bis C₄)-alkylgruppen substituiert ist,
v) mittels Triethoxycaprylsilan beschichtete Metalloxidpartikel, die keine fluorierten organischen Gruppen tragen enthält.

Die Kern-Hülle-Teilchen der pulverförmigen Zusammensetzung gemäß dieser bevorzugten Ausführungsform umfassen folglich einen Kern, der eine wässrige, flüssige Phase umfasst. Der Kern liegt somit in flüssiger Form vor. Diesen Kern umgibt eine Hülle, die auf separierbaren einzelnen Partikeln von an der Oberfläche mit fluorhaltigen, organischen Gruppen beschichtetem Metalloxid basiert.

Die erfindungsgemäßen pulverförmigen Zusammensetzungen als zwingend erforderliche Komponente 50 bis 90 Gew.-%, insbesondere 60 bis 85 Gew.-%, Wasser, bezogen auf das Gewicht der pulverförmigen Zusammensetzung.

Ferner enthalten die erfindungsgemäßen pulverförmigen Zusammensetzungen als weitere zwingend erforderliche Komponente 0,5 Gew.-% bis 10 Gew.-% mindestens einer weiteren Flüssigkeit, die einen Siedepunkt von mehr als 150°C bei einem Druck von 1013 mbar aufweist. Im Rahmen einer bevorzugten Ausführungsform liegen die eingesetzten weiteren Flüssigkeiten mit einen Siedepunkt von mehr als 150°C bei einem Druck von 1013 mbar, bei 20°C in Wasser gelöst vor.

Es ist wiederum besonders bevorzugt, wenn diese weitere Flüssigkeit mit einem Siedepunkt von mehr als 150°C bei einem Druck von 1013 mbar aus mindestens einem (C₃ bis C₈)-Alkohol mit mindestens 2 Hydroxylgruppen ausgewählt wird.

Ganz besonders bevorzugt wird diese weitere Flüssigkeit mit einem Siedepunkt von mehr als 100°C bei einem Druck von 1013 mbar aus mindestens einer Verbindung aus der Gruppe ausgewählt, bestehend aus Glyzerin, 1,3-Butylenglykol, 1,4-Butylenglykol, 1,2-Pentandiol, 2-Methyl-2,4-dihydroxypentan und 2-Ethyl-1,3-hexandiol. Am bevorzugtesten enthält die erfindungsgemäße pulverförmige Zusammensetzung als weitere Flüssigkeit mit einem Siedepunkt von mehr als 150°C bei einem Druck von 1013 mbar Glyzerin.

Als weitere zwingend erforderliche Komponente enthält die erfindungsgemäße pulverförmige Zusammensetzung mindestens eine mit Carboxy-(C₁ bis C₄)-alkylgruppen substituierte Stärke. Die mit Carboxy-(C₁ bis C₄)-alkylgruppen substituierte Stärke kann erfindungsgemäß ebenso in neutralisierter Form ihrer Salze, beispielsweise als Natriumsalz, Kaliumsalz eingesetzt werden. Die mit Carboxy-(C₁ bis C₄)-alkylgruppen substituierte Stärke ist in den erfindungsgemäßen pulverförmigen Zusammensetzungen bevorzugt in einer Menge von 0,1 Gew.-% bis 5 Gew.-%, insbesondere 0,75 Gew.-% bis 3,5 Gew.-%, jeweils bezogen auf das Gewicht der pulverförmigen Zusammensetzung, enthalten.

Im Rahmen einer erfindungsgemäß bevorzugten Ausführungsform enthält die pulverförmige Zusammensetzung als mit Carboxy-(C₁ bis C₄)-alkylgruppen substituierte Stärke, mindestens eine Verbindung ausgewählt aus der Gruppe bestehend aus, Carboxymethylstärke, Carboxyethylstärke, Carboxypropylstärke. Dabei eignet sich wiederum die Carboxymethylstärke ganz besonders bevorzugt zum Einsatz in den erfindungsgemäßen pulverförmigen Zusammensetzungen. Carboxymethylstärke wird beispielsweise in Form des Handelsprodukts Covagel (Carboxymethylstärke, Natriumsalz, als Stärkequelle diente Kartoffelstärke; INCI-Bezeichnung: Sodium Carboxymethyl Starch) der Firma Sensient /LCW eingesetzt.

Die erfindungsgemäße pulverförmigen Zusammensetzung enthält ferner als zwingend erforderlichen Bestandteil Metalloxidpartikel, die an der Oberfläche mit fluorhaltigen, organischen Gruppen beschichtet wurden. Definitionsgemäß versteht der Fachmann unter fluorhaltigen organischen Gruppen solche chemischen Moleküle/Molekülfragmente, die mindestens ein Kohlenstoffatom und mindestens ein daran kovalent gebundenes Fluoratom umfassen. Diese fluorierten organischen Gruppen können im Sinne der Erfindung kovalent an die Oberfläche des Metalloxidpartikels gebunden sein oder (z.B. in Form eines Öls) an der Oberfläche des Metalloxidpartikels adsorbiert sein.

Die erfindungsgemäß pulverförmige Zusammensetzung enthält die an der Oberfläche mit fluorhaltigen, organischen Gruppen beschichteten Metalloxidpartikel bevorzugt in einer Menge von 5 Gew.-% bis 25 Gew.-% - bezogen auf das Gewicht der gesamten pulverförmigen Zusammensetzung.

Ferner hat es sich als vorteilhaft erwiesen, wenn die Metalloxidpartikel, die an der Oberfläche mit fluorhaltigen, organischen Gruppen beschichtet wurden einen Partikeldurchmesser von weniger als 5 µm, bevorzugt weniger als 1 µm, besonders bevorzugt zwischen 20 und 100 nm, aufweisen Die an der Oberfläche mit fluorhaltigen, organischen Gruppen beschichteten Metalloxidpartikel werden bevorzugt ausgewählt aus einem Vertreter der Gruppe, die gebildet wird aus mit fluorhaltigen, organischen Gruppen beschichteten Aluminaten, mit fluorhaltigen, organischen Gruppen beschichteten Silikaten, mit fluorhaltigen, organischen Gruppen beschichteten Aluminiumsilikaten, mit fluorhaltigen, organischen Gruppen beschichtetem Titandioxid sowie mit fluorhaltigen, organischen Gruppen beschichtetem Silikagel.

Besonders bevorzugte mit fluorhaltigen, organischen Gruppen beschichtete Aluminate werden ausgewählt unter aktivem Aluminiumoxid, alpha-Aluminiumoxid, beta-Aluminiumoxid, gamma-Aluminiumoxid sowie deren Gemischen, wobei alle genannten Aluminate entsprechend mit fluorhaltigen, organischen Gruppen beschichtetet sind.

Besonders bevorzugte mit fluorhaltigen, organischen Gruppen beschichtete Aluminiumsilikate (auch Alumosilikate genannt) werden ausgewählt unter Phyllosilikaten, Tectosilikaten, wobei alle genannten Aluminiumsilikate entsprechend mit fluorhaltigen, organischen Gruppen beschichtet sind.

Bevorzugt geeignete mit fluorhaltigen, organischen Gruppen beschichtete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonit, Phlogopit, Biotit, Lepidolith, Margarit, Smektit (hier insbesondere unter Montmorrilionit, Saponit, Nontronit, Hectorit)), wobei alle genannten Phyllosilikate entsprechend mit fluorhaltigen, organischen Gruppen beschichtetet sind.

Bevorzugt geeignete mit fluorhaltigen, organischen Gruppen beschichtete Tectosilikate werden ausgewählt unter Feldspatmineralien (insbesondere Albit, Orthoklas, Anorthit, Leucit, Sodalith, Hauyn, Labradorit, Lasurit, Nosean, Nephelin), wobei alle genannten Tectosilikate entsprechend mit fluorhaltigen, organischen Gruppen beschichtetet sind.

Dabei ist es wiederum besonders bevorzugt, wenn das besagte Metalloxidpartikel ausgewählt werden, aus mindestens einem Vertreter der Gruppe die gebildet wird aus an der Oberfläche mit fluorhaltigen, organischen Gruppen beschichtetem Phyllosilikat (insbesondere an der Oberfläche mit fluorhaltigen, organischen Gruppen beschichtetem Talk, an der Oberfläche mit fluorhaltigen, organischen Gruppen beschichtetem Glimmer), an der Oberfläche mit fluorhaltigen, organischen Gruppen beschichtetem Titandioxid, an der Oberfläche mit fluorhaltigen, organischen Gruppen beschichtetem Siliziumdioxid.

Besonders zur Ausführung der Erfindung geeignete an der Oberfläche mit fluorhaltigen, organischen Gruppen beschichtete Metalloxidpartikel sind solche, die an der Oberfläche mit pertluorierten organischen Verbindungen, insbesondere mit Perfluor-alkylresten, beschichtet wurden. Dabei sind wiederum solche besagten Metalloxide bevorzugt, die mit mindestens einem Rest hydrophobiert wurden, ausgewählt aus Pertluoralkylsilyl, Polyperfluor-(C₂ bis C₆)-alkylenoxid, Polysiloxan mit Perfluoralkylgruppen, Perfluoralkylphosphat, Polyfluoralkylphosphatether. Darunter werden solche an der Oberfläche mit fluorhaltigen, organischen Gruppen beschichteten Metalloxidpartikel bevorzugt verwendet, die an der Oberfläche Polyperfluor-(C₂ bis C₆)-alkylenoxidreste tragen. Diese werden bevorzugt ausgewählt aus mit Polyperfluor-(C₂ bis C₆)-alkylenoxid beschichtetem Glimmer und/oder mit Polyperfluor-(C₂ bis C₆)-alkylenoxid beschichtetem Titandioxid und/oder mit Polyperfluor-(C₂ bis C₆)-alkylenoxid beschichtetem Siliziumdioxid und/oder mit Polyperfluor-(C₂ bis C₆)-alkylenoxid beschichtetem Talk.

Besonders bevorzugt einsetzbar sind dabei die Handelsprodukte PW Covafluor^{®} (Firma LCW), PFS Talc JA 46-R^{®} (Firma Daito), Talc JA R46PF^{®} (Firma LCW), Submica M^{®} (Firma LCW).

Ganz besonders bevorzugt enthält die erfindungsgemäße pulverförmige Zusammensetzung solche an der Oberfläche mit fluorhaltigen, organischen Gruppen beschichtete Metalloxidpartikel, die an der Oberfläche durch Umsetzung mit mindestens einem Reagenz der Formel (I) beschichtet wurden 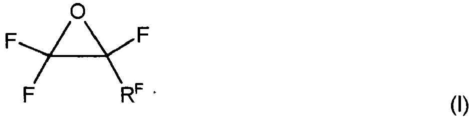 worin R^{F} für eine Perfluor-(C₁ bis C₄)-alkylgruppe, insbesondere Trifluormethyl, steht. Metalloxidpartikel, die mit Reagenzien der Formel (I) beschichtet wurden erhalten kovalent an der Oberfläche gebundene Polyperfluor-(C₂ bis C₆)-alkylenoxidreste.

Darüber hinaus ist es erfindungsgemäß bevorzugt, wenn die erfindungsgemäße pulverförmige Zusammensetzung solche an der Oberfläche mit fluorhaltigen, organischen Gruppen beschichteten Metalloxidpartikel enthält, die an der Oberfläche durch mindestens eine Verbindung der Formel (la) beschichtet sind 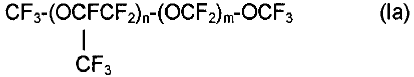 worin die Summe n + m für eine ganze Zahl von 20 bis 80 steht. Diese Verbindungen besitzen die INCI-Bezeichnung Polyperfluoromethylisopropyl Ether. Besonders bevorzugt eignen sich Verbindungen der Formel (la), die ein Molekulargewicht von 1500 bis 4000 g/mol aufweisen.

Ganz besonders bevorzugt einsetzbar ist das Handelsprodukt PW F-MS (mit Polyperfluoromethylisopropylether (CAS-Nr.: 69991-67-9, EINECS 274-225-4) und Triethoxycaprylylsilan beschichtetes, nanofeines Titandioxid (INCI-Bezeichnung: CI 77891, Polyperfluoromethylisopropyl Ether, Triethoxycaprylylsilane) der Firma Sensient / LCW.

Es hat sich als erfindungsgemäß vorteilhaft erwiesen, den erfindungsgemäßen pulverförmigen Zusammensetzungen - zusätzlich zu den an der Oberfläche mit fluorhaltigen, organischen Gruppen beschichteten Metalloxidpartikeln - silanisierte Metalloxidpatikel zuzusetzen, die keine fluorierten organischen Gruppen tragen.

Die erfindungsgemäße pulverförmige Zusammensetzung dieser Ausführungsform umfasst bevorzugt Kern-Hülle-Teilchen, deren Hülle
(a) an der Oberfläche mit fluorhaltigen, organischen Gruppen beschichtete Metalloxidpartikel (insbesondere diejenigen der vorgenannten bevorzugten Ausführungsformen) und
(b) silanisierte Metalloxidpatikel (insbesondere diejenigen der nachfolgend genannten bevorzugten Ausführungsformen). Diese Beschichtung enthält Triethoxycaprylsilan
enthält und deren Kern eine flüssige, wässrige Phase umfasst, dadurch gekennzeichnet, dass die pulverförmige Zusammensetzung
i) 50 bis 90 Gew.-% Wasser,
ii) 0,5 Gew.-% bis 10 Gew.-% mindestens einer weiteren Flüssigkeit, die einen Siedepunkt von mehr als 150°C bei einem Druck von 1013 mbar aufweist,
iii) mindestens eine Stärke, die mit Carboxy-(C₁ bis C₄)-alkylgruppen substituiert ist,
enthält.

Die Kern-Hülle-Teilchen der pulverförmigen Zusammensetzung gemäß dieser bevorzugten Ausführungsform umfassen folglich einen Kern, der eine wässrige, flüssige Phase umfasst. Der Kern liegt somit in flüssiger Form vor. Diesen Kern umgibt eine Hülle, die auf separierbaren einzelnen Partikeln von an der Oberfläche mit fluorhaltigen, organischen Gruppen beschichtetem Metalloxid und von silanisiertem Metalloxid basiert.

Die silanisierten Metalloxidpartikel werden bevorzugt ausgewählt aus silanisierten Aluminaten, silanisierten Silikaten, silanisierten Aluminiumsilikaten, silanisiertem Titandioxid sowie silanisiertem Silikagel.

Besonders bevorzugte silanisierte Aluminate werden ausgewählt unter aktivem silanisiertem Aluminiumoxid, silanisiertem alpha-Aluminiumoxid, silanisiertem beta-Aluminiumoxid, silanisiertem gamma-Aluminiumoxid sowie deren Gemischen.

Besonders bevorzugte silanisierte Aluminiumsilikate (auch Alumosilikate genannt) werden ausgewählt unter silanisierten Phyllosilikaten, silanisierten Tectosilikaten.

Bevorzugt geeignete silanisierte Phyllosilikate werden ausgewählt unter silanisierten Kaolinen (hier insbesondere unter silanisiertem Kaolinit, silanisiertem Dickit, silanisiertem Hallosit sowie silanisiertem Nakrit), silanisiertem Serpentin, silanisiertem Talk, silanisiertem Pyrophyllit, silanisiertem Montmorillonit, silanisiertem Quarz, silanisiertem Bentonit, silanisiertem Glimmer (hier insbesondere unter silanisiertem Illit, silanisiertem Muscovit, silanisiertem Paragonit, silanisiertem Phlogopit, silanisiertem Biotit, silanisiertem Lepidolith, silanisiertem Margarit, silanisiertem Smektit (hier insbesondere unter silanisiertem Montmorrilionit, silanisiertem Saponit, silanisiertem Nontronit, silanisiertem Hectorit)).

Bevorzugt geeignete silanisierte Tectosilikate werden ausgewählt unter silanisierten Feldspatmineralien (insbesondere silanisiertem Albit, silanisiertem Orthoklas, silanisiertem Anorthit, silanisiertem Leucit, silanisiertem Sodalith, silanisiertem Hauyn, silanisiertem Labradorit, silanisiertem Lasurit, silanisiertem Nosean, silanisiertem Nephelin).

Die zusätzlichen silanisierten Metalloxidpartikel weisen bevorzugt einen Partikeldurchmesser von weniger als 5 µm, bevorzugt weniger als 1 µm, besonders bevorzugt zwischen 20 und 100 nm, auf.

Im Rahmen der Ausführungsform ist es besonders bevorzugt, wenn die zusätzlich in der erfindungsgemäßen pulverförmige Zusammensetzung enthaltenen silanisierten Metalloxidpartikel ausgewählt werden aus Metalloxidpartikeln, die durch Umsetzung mit mindestens einem Reagens der Formel (II) beschichtet wurden

(R¹O)ₙO(CH₃)₃₋ₙSi-R² (II)

worin
R¹ für Methyl oder Ethyl steht,
R² für eine (C₁ bis C₁₄)-Alkylgruppe, insbesondere eine (C₆ bis C₁₂)-Alkylgruppe, steht,
n für 1, 2 oder 3 steht.

Dabei sind solche Reagenzien der Formel (II) bevorzugt, in denen n die Zahl 3 bedeutet.

Insbesondere ist es erfindungsgemäß besonders bevorzugt, wenn besagte zusätzliche Metalloxidpartikel ausgewählt werden, aus mindestens einem Vertreter der Gruppe die gebildet wird aus an der Oberfläche durch Umsetzung mit mindestens einem Reagens der Formel (II) beschichtetem Phyllosilikat (insbesondere an der Oberfläche durch Umsetzung mit mindestens einem Reagens der Formel (II) beschichtetem Talk, an der Oberfläche durch Umsetzung mit mindestens einem Reagens der Formel (II) beschichtetem Glimmer), an der Oberfläche durch Umsetzung mit mindestens einem Reagens der Formel (II) beschichtetem Titandioxid, an der Oberfläche durch Umsetzung mit mindestens einem Reagens der Formel (II) beschichtetem Siliziumdioxid.

Die zusätzlichen Metalloxidpartikel wurden durch Umsetzung mit Triethoxycaprylylsilan beschichtet. Dabei ist es wiederum besonders bevorzugt, wenn das besagte Metalloxidpartikel ausgewählt werden, aus mindestens einem Vertreter der Gruppe die gebildet wird aus an der Oberfläche durch Umsetzung durch Umsetzung mit Triethoxycaprylylsilan beschichtetem Phyllosilikat (insbesondere an der Oberfläche durch Umsetzung durch Umsetzung mit Triethoxycaprylylsilan beschichtetem Talk, an der Oberfläche durch Umsetzung durch Umsetzung mit Triethoxycaprylylsilan beschichtetem Glimmer), an der Oberfläche durch Umsetzung durch Umsetzung mit Triethoxycaprylylsilan beschichtetem Titandioxid, an der Oberfläche durch Umsetzung mit Triethoxycaprylylsilan beschichtetem Siliziumdioxid. Solche Metalloxidpartikel werden beispielsweise unter den Handelsnamen Mica 8 AS R0433 (durch Umsetzung mit Triethoxycaprylylsilan beschichteter Glimmer aus 99% Mica, 1 % Triethoxycaprylylsilan; INCI-Bezeichnung: Mica, Triethoxycaprylylsilan), Covapearl Bright 933 AS (mit Triethoxycaprylylsilan beschichtete mineralische Perlglanzpigmente aus 25-35% Titandioxid, 65-75% Mica, 2% Triethoxycaprylylsilan; INCI-Bezeichnung: CI 77891, Mica, Triethoxycaprylylsilane) (Sensient / LCW), PW Covasil S (INCI-Bezeichnung: Titanium Dioxide, Trimethoxycaprylylsilane, Polymethyl methacrylate), Talc AS R0435 (durch Umsetzung mit Triethoxycaprylylsilan beschichteter Talk aus 99% Talk und 1 % Triethoxycaprylylsilan; INCI-Bezeichnung: Talc, Triethoxycaprylylsilane) jeweils von der Firma Sensient / LCW angeboten.

Besonders bevorzugt enthält die erfindungsgemäße pulverförmige Zusammensetzung solche Metalloxidpartikel, die an der Oberfläche durch Umsetzung mit mindestens einem Reagenz der obigen Formel (I) und mindestens einem Reagenz der obigen Formel (II) beschichtet wurden. Hier befinden sich - in Abgrenzung zum zuvor beschriebenen bloßen Zusatz silanisierter Metalloxidpartikel zu den mit fluorierten, organischen Gruppen beschichteten Metalloxidpartikeln - die besagten Beschichtungen gemeinsam auf ein und demselben Metalloxidpartikel.

es ist erfindungsgemäß bevorzugt, dass die zusätzlichen silanisierten Metalloxidpartikel und die an der Oberfläche mit fluorhaltigen, organischen Gruppen beschichteten Metalloxidpartikel insgesamt in einer Menge von 5 Gew.-% bis 25 Gew.-% (bezogen auf das Gewicht der pulverförmigen Zusammensetzung) enthalten sind.

Die erfindungsgemäße pulverförmige Zusammensetzung kann zur Steigerung des Frisurenhaltes zusätzlich mindestens ein filmbildendes und/oder festigendes Polymer enthalten.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigem, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers werden häufig der so genannte curl-retention - Test oder der Dreipunkt-Biegetest angewendet.

Die erfindungsgemäße pulverförmige Zusammensetzung enthält bevorzugt als filmbildendes und/oder festigendes Polymer
- mindestens ein kationisches filmbildendes und/oder kationisches festigendes Polymer und/oder
- mindestens ein nichtionisches filmbildendes und/oder nichtionisches festigendes Polymer und/oder
- mindestens ein anionisches filmbildendes und/oder anionisches festigendes Polymer und/oder
- mindestens ein amphoteres filmbildendes und/oder amphoteres festigendes Polymer.

Im Rahmen einer weiteren Ausführungsform enthalten die erfindungsgemäßen pulverförmigen Zusammensetzungen als filmbildendes und/oder festigendes Polymer mindestens ein kationisches filmbildendes,und/oder kationisches festigendes Polymer.

Die kationischen filmbildenden und/oder kationischen festigenden Polymere weisen mindestens eine Struktureinheit auf, die mindestens ein permanent kationisiertes Stichstoffatom enthält. Unter permanent kationisierten Stickstoffatomen sind solche Stickstoffatome zu verstehen, die eine positive Ladung tragen und dadurch eine quartäre Ammoniumverbindung bilden. Quartäre Ammonium-Verbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen.

Im Sinne der Erfindung bevorzugte pulverförmige Zusammensetzungen enthalten die filmbildenden kationischen und/oder festigenden kationischen Polymere in einer Menge von 0,1 Gew.-% bis 8,0 Gew.-%, besonders bevorzugt von 0,2 Gew.-% bis 7,0 Gew.-%, ganz besonders bevorzugt von 0,5 Gew.-% bis 5,0 Gew.-%, jeweils bezogen auf das Gewicht der Zusammensetzung.

Die kationischen filmbildenden und/oder kationischen festigenden Polymere können erfindungsgemäß aus kationischen, quaternisierten Cellulose-Derivaten ausgewählt werden.

Es erweisen sich generell solche kationischen, quaternisierten Cellulosen als im Sinne der Erfindung vorteilhaft, die in einer Seitenkette mehr als eine permanente kationische Ladung tragen.

Darunter sind unter den kationischen Cellulosederivaten solche hervorzuheben, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Weiterhin eignen sich solche kationischen filmbildenden und/oder kationischen festigenden Polymere, die mindestens eine Struktureinheit der Formel (M-I) und mindestens eine Struktureinheit der Formel (M-VI) und gegebenenfalls mindestens eine Struktureinheit der Formel (M-V) umfassen 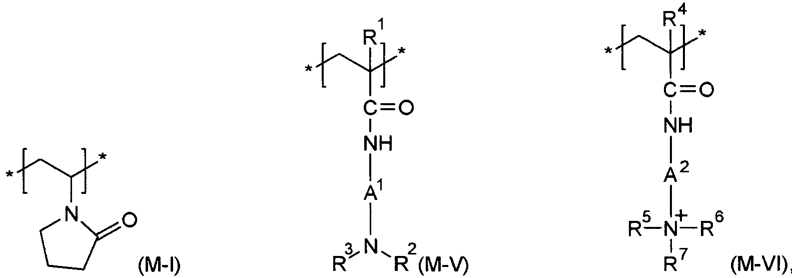 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

Zur Kompensation der positiven Ladung des Monomers (M-VI) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Geeignete Verbindungen sind beispielsweise als
- Copolymere aus mit Diethylsulfat quaterniertem Dimethylaminoethylmethacrylat, mit Vinylpyrrolidon mit der INCI-Bezeichnung Polyquatemium-11 unter den Bezeichnungen Gafquat^{®} 440, Gafquat^{®}734, Gafquat^{®}755 (jeweils Firma ISP) sowie Luviquat PQ 11 PN (Firma BASF SE),
- Copolymere aus Methacryloylaminopropyllauryldimethylammonium chlorid mit Vinylpyrrolidon und Dimethylaminopropylmethacrylamid mit der INCI-Bezeichnung Polyquaternium-55 unter den Handelsnamen, Styleze^{®} W-10, Styleze^{®} W-20 (Firma ISP),
im Handel erhältlich.

Als im Sinne der Erfindung besonders bevorzugt verwendbare filmbildende und/oder festigende Polymere ausgewählt aus kationischen Polymeren die mindestens eine Struktureinheit enthalten, die ein permanent kationisiertes Stickstoffatom aufweisen, dienen weiterhin solche kationischen filmbildenden und/oder kationischen festigenden Polymere, die mindestens ein Strukturelement der Formel (M1) enthalten 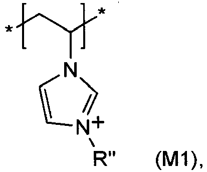 worin
R" für eine (C₁ bis C₄)-Alkylgruppe, insbesondere eine Methylgruppe, steht,
und zusätzlich mindestens ein weiteres kationisches und/oder nichtionisches Strukturelement aufweisen.

Zur Kompensation der positiven Polymerladung der Komponente dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum erfindungsgemäß bevorzugt, wenn in der erfindungsgemäßen pulverförmigen Zusammensetzung als kationisches filmbildendes und/oder kationisches festigendes Polymer dieser Ausführungsform, mindestens ein Copolymer (b1) enthalten ist, das neben mindestens einem Strukturelement der Formel (M1) zusätzlich ein Strukturelement der Formel (M-I) umfasst 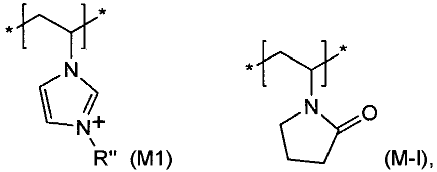 worin
R" für eine (C₁ bis C₄)-Alkylgruppe, insbesondere eine Methylgruppe, steht.

Zur Kompensation der positiven Polymerladung der Copolymere (b1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (b1) enthalten 10 bis 30 Mol-%, vorzugsweise 15 bis 25 Mol.-% und insbesondere 20 Mol.-% Struktureinheiten gemäß Formel (M1) und 70 bis 90 Mol.-%, vorzugsweise 75 bis 85 Mol.-% und insbesondere 80 Mol.% Struktureinheiten It. Formel (M-I). Hierbei ist besonders bevorzugt, wenn die Copolymere (b1) neben Polymereinheiten, die aus dem Einbau der genannten Struktureinheiten gemäß Formel (M1) und (M-I) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b1) ausschließlich aus Struktureinheiten der Formel (M1) mit R" = Methyl und (M-I) aufgebaut und lassen sich durch die allgemeine Formel (Poly1) 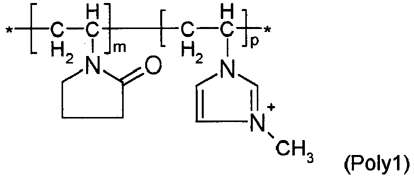 beschreiben, wobei die Indices m und p je nach Molmasse des Polymers variieren und nicht bedeuten sollen, daß es sich um Blockcopolymere handelt. Vielmehr können Struktureinheiten der Formel (M1) und der Formel (M-I) im Molekül statistisch verteilt vorliegen. Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich.

Besonders bevorzugte erfindungsgemäße pulverförmige Zusammensetzungen enthalten ein Copolymer (b1), insbesondere der Formel (Poly1), das Molmassen innerhalb eines bestimmten Bereiches aufweist. Hier sind erfindungsgemäße Mittel bevorzugt, bei denen das Copolymer (b1) eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) (b1) oder an dessen bzw. deren Stelle können erfindungsgemäße pulverförmige Zusammensetzungen auch Copolymere (b2) enthalten, die ausgehend vom Copolymer (b1) als zusätzliche Struktureinheiten Struktureinheiten der Formel (M-II) aufweisen 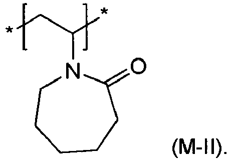 Weitere besonders bevorzugte erfindungsgemäße pulverförmige Zusammensetzungen sind somit dadurch gekennzeichnet, daß sie als kationisches filmbildendes und/oder kationisches festigendes Polymer mindestens ein Copolymer (b2) enthalten, das mindestens eine Struktureinheit gemäß Formel (M1-a) und mindestens eine Struktureinheit gemäß Formel (M-I) und mindestens eine Struktureinheit gemäß Formel (M-II) enthält

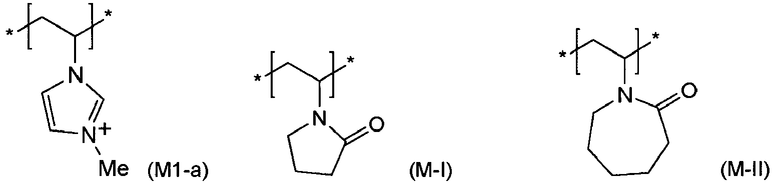

Auch hierbei ist besonders bevorzugt, wenn die Copolymere (b2) neben Polymereinheiten, die aus dem Einbau der genannten Struktureinheiten gemäß Formel (M1-a), (M-I) und (M-II) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b2) ausschließlich aus Struktureinheiten der Formeln (M1-a), (M-I) und (M-II) aufgebaut und lassen sich durch die allgemeine Formel (Poly2) 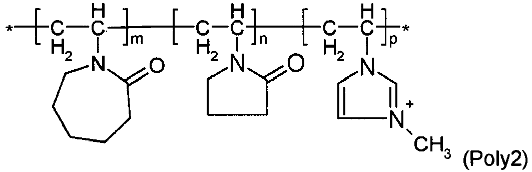 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen. Zur Kompensation der positiven Polymerladung der Komponente (b2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (b2) enthalten 1 bis 20 Mol-%, vorzugsweise 5 bis 15 Mol.-% und insbesondere 10 Mol.% Struktureinheiten gemäß Formel (M1-a) und 30 bis 50 Mol.-%, vorzugsweise 35 bis 45 Mol.-% und insbesondere 40 Mol.-% Struktureinheiten gemäß Formel (I) und 40 bis 60 Mol.-%, vorzugsweise 45 bis 55 Mol.-% und insbesondere 60 Mol.-% Struktureinheiten gemäß Formel (M-II).

Besonders bevorzugte erfindungsgemäße pulverförmige Zusammensetzungen enthalten ein Copolymer (b2), das Molmassen innerhalb eines bestimmten Bereiches aufweist. Hier sind erfindungsgemäße Mittel bevorzugt, bei denen das Copolymer (b2) eine Molmasse von 100 bis 1000 kDa, vorzugsweise von 250 bis 900 kDa, weiter bevorzugt von 500 bis 850 kDa und insbesondere von 650 bis 710 kDa aufweist.

Zusätzlich zu dem bzw. den Copolymer(en) (b1) und/oder (b2) oder an dessen bzw. deren Stelle können die erfindungsgemäßen pulverförmigen Zusammensetzungen als filmbildendes kationische und/oder festigendes kationisches Polymer auch Copolymere (b3) enthalten, die als Struktureinheiten Struktureinheiten der Formeln (M1-a) und (I) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße pulverförmige Zusammensetzungen sind dadurch gekennzeichnet, daß sie als kationisches filmbildendes und/oder kationisches festigendes Polymer mindestens ein Copolymer (b3) enthalten, das mindestens eine Struktureinheit gemäß Formel (M1-a) und mindestens eine Struktureinheit gemäß Formel (M-I) und mindestens eine Struktureinheit gemäß Formel (M-VII) und mindestens eine Struktureinheit gemäß Formel (M-VIII) enthält

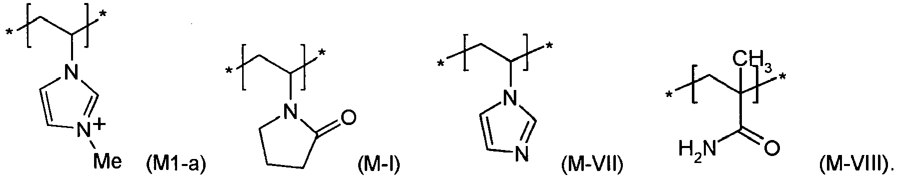

Auch hierbei ist besonders bevorzugt, wenn die Copolymere (b3) neben Polymereinheiten, die aus dem Einbau der genannten Struktureinheiten gemäß Formel (M1-a), (M-I), (M-VII) und (M-VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b3) ausschließlich aus Struktureinheiten der Formel (M1-a), (M-I), (M-VII) und (M-VIII) aufgebaut und lassen sich durch die allgemeine Formel (Poly3) 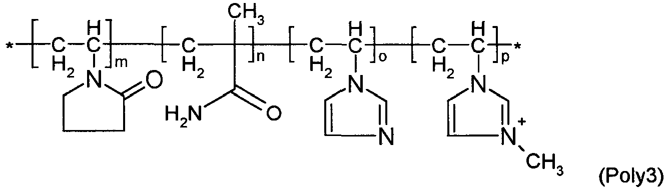 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M1-a), (M-I), (M-VII) und (M-VIII) im Molekül statistisch verteilt vorliegen.

Zur Kompensation der positiven Polymerladung der Komponente (b2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (b3) enthalten 1 bis 12 Mol-%, vorzugsweise 3 bis 9 Mol.-% und insbesondere 6 Mol.-% Struktureinheiten gemäß Formel (M1-a) und 45 bis 65 Mol.-%, vorzugsweise 50 bis 60 Mol.-% und insbesondere 55 Mol.-% Struktureinheiten gemäß Formel (M-I) und 1 bis 20 Mol.-%, vorzugsweise 5 bis 15 Mol.-% und insbesondere 10 Mol.-% Struktureinheiten gemäß Formel (M-VII) und 20 bis 40 Mol.-%, vorzugsweise 25 bis 35 Mol.-% und insbesondere 29 Mol.-% Struktureinheiten gemäß Formel (M-VIII).

Besonders bevorzugte erfindungsgemäße Mittel enthalten ein Copolymer (b3), das Molmassen innerhalb eines bestimmten Bereiches aufweist. Hier sind erfindungsgemäße Mittel bevorzugt, bei denen das Copolymer (b3) eine Molmasse von 100 bis 500 kDa, vorzugsweise von 150 bis 400 kDa, weiter bevorzugt von 250 bis 350 kDa und insbesondere von 290 bis 310 kDa aufweist. Unter den zusätzlichen filmbildenden kationischen und/oder festigenden Polymer ausgewählt aus den kationischen Polymeren mit mindesten einem Strukturelement der obigen Formel (M1), gelten als bevorzugt:
- Vinylpyrrolidon/1-Vinyl-3-methyl-1H-imidazoliumchlorid-Copolymere,
- Vinylpyrrolidon/1-Vinyl-3-methyl-1H-imidazoliummethylsulfat-Copolymere,
- Vinylpyrrolidon/Vinylcaprolactam/1-Vinyl-3-methyl-1H-imidazolium-Terpolymer,
- Vinylpyrrolidon/Methacrylamid/Vinylimidazol/1-Vinyl-3-methyl-1H-imidazoliummethylsulfat-Copolymer, sowie Gemische aus diesen Polymeren.

Im Rahmen einer bevorzugten Ausführungsform enthalten die erfindungsgemäßen pulverförmigen Zusammensetzungen als filmbildendes und/oder festigendes Polymer mindestens ein filmbildendes nichtionisches und/oder festigendes nichtionisches Polymer.

Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die filmbildenden nichtionischen und/oder festigenden nichtionischen Polymere sind in dem erfindungsgemäßen Mittel bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 5,0 Gew.-%, jeweils bezogen auf das Gewicht der erfindungsgemäßen pulverförmigen Zusammensetzung, enthalten.

Die filmbildenden nichtionischen und/oder festigenden nichtionischen Polymere werden wiederum bevorzugt ausgewählt aus mindestens einem Polymer der Gruppe, die gebildet wird, aus
- Homopolymeren und nichtionischen Copolymeren des N-Vinylpyrrolidons,
- nichtionischen Copolymeren des Isobutens.

Geeignete Polyvinylpyrrolidone sind beispielsweise Handelsprodukte wie Luviskol^{®} K 90 oder Luviskol^{®} K 85 der Firma BASF SE.

Geeignete Polyvinylalkohole werden beispielsweise unter den Handelsbezeichnungen Elvanol^{®} von Du Pont oder Vinol^{®} 523/540 von der Firma Air Products vertrieben.

Geeignetes Polyvinylacetat wird beispielsweise unter dem Handelsnamen Vinac^{®} als Emulsion von der Firma Air Products vertrieben.

Mittel, die als filmbildendes nichtionisches und/oder festigendes nichtionisches Polymer mindestens ein Polymer ausgewählt aus der Gruppe, die gebildet wird aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
- Copolymere aus N-Vinylpyrrolidon mit N,N-Di(C₁ bis C₄)-Alkylamino-(C₂ bis C₄)-alkylacrylamid,
sind erfindungsgemäß ganz besonders bevorzugt.

Dabei ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt.

Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Weitere bevorzugte erfindungsgemäße pulverförmige Zusammensetzungen sind dadurch gekennzeichnet, daß sie als nichtionisches filmbildendes und/oder nichtionisches festigendes Polymer mindestens ein Copolymer (n1) enthalten, das
- mindestens weitere Struktureinheit gemäß Formel (M-I) enthält 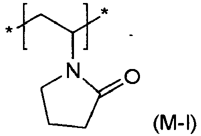
- mindestens weitere Struktureinheit gemäß Formel (M-VII) enthält 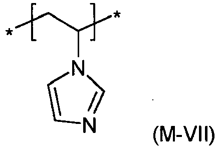
- mindestens weitere Struktureinheit gemäß Formel (M-VIII) enthält

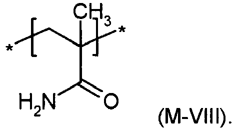

Auch hierbei ist besonders bevorzugt, wenn diese Copolymere neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (n1) ausschließlich aus Struktureinheiten der Formel (M1-a), (I), (VII) und (VIII) aufgebaut und lassen sich durch die allgemeine Formel (Poly4) 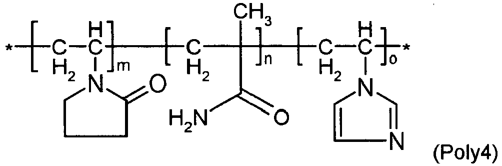 beschreiben, wobei die Indices m, n, o und p je nach Molmasse des Polymers variieren und nicht bedeuten sollen, daß es sich um Blockcopolymere handelt. Vielmehr können Struktureinheiten der Formeln (I), (VII) und (VIII) im Molekül statistisch verteilt vorliegen.

Ein besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Weiterhin eignen sich erfindungsgemäß solche pulverförmigen Zusammensetzungen, die mindestens ein nichtionisches filmbildendes und/oder nichtionisches festigendes Polymer enthalten, umfassend mindestens eine Struktureinheit der Formel (M-I) und mindestens eine Struktureinheit der Formel (M-III) 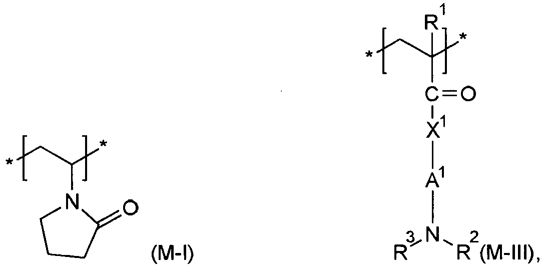 worin
R¹ für ein Wasserstoffatom oder eine Methylgruppe steht,
X¹ für ein Sauerstoffatom oder eine Gruppe NH steht,
A¹ für eine Gruppe Ethan-1,2-diyl, Propan-1,3-diyl oder Butan-1,4-diyl steht
R² und R³ stehen unabhängig voneinander für eine (C₁ bis C₄)-Alkylgruppe.

Dabei ist es insbesondere bevorzugt, wenn das obige nichtionische filmbildende und/oder nichtionische festigende Polymer ausgewählt wird aus mindestens einem Polymer, welches mindestens eines oder mehrere der folgenden Merkmale erfüllt:
- R¹ bedeutet eine Methylgruppe,
- X¹ steht für eine Gruppe NH,
- A¹ steht für Ethan-1,2-diyl oder Propan-1,3-diyl,
- R² und R³, stehen unabhängig voneinander für Methyl oder Ethyl, (besonders bevorzugt für Methyl).

Besonders bevorzugt handelt es sich bei dem nichtionischen filmbildenden und/oder nichtionischen festigenden Polymer dieser Ausführungsform um mindestens ein Polymer, das mindestens eine Struktureinheit der Formel (M-I) und mindestens eine Struktureinheit der Formel (M-III-8) umfasst, 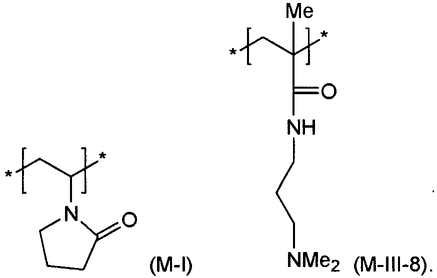 Ein ganz besonders bevorzugtes nichtionisches filmbildendes und/oder nichtionisches festigendes Polymer dieser Ausführungsform ist ein Copolymer aus N-Vinylpyrrolidon und N,N-Dimethylaminiopropylmethacrylamid, welches beispielsweise mit der INCI-Bezeichnung VP/DMAPA Acrylates Copolymer z.B. unter der Handelsbezeichnung Styleze^{®} CC 10 von der Firma ISP verkauft wird.

Im Rahmen einer bevorzugten Ausführungsform enthalten die erfindungsgemäßen Zusammensetzungen als filmbildendes und/oder festigendes Polymer mindestens ein filmbildendes anionisches und/oder festigendes anionisches Polymer.

Unter einem anionischen Polymer wird erfindungsgemäß ein Polymer verstanden, das in einem protischen Lösemittel bei Standardbedingungen Struktureinheiten mit anionischen Gruppen, welche durch Gegenionen unter Erhaltung der Elektroneutralität kompensiert werden müssen, trägt und keine Struktureinheiten mit permanent kationischen oder kationisierbaren Gruppen aufweist. Unter anionische Gruppen fallen Carboxyl- und Sulfonsäuregruppen.

Die anionischen, filmbildenden und/oder anionischen, festigenden Polymere sind in dem erfindungsgemäßen Mittel bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht der erfindungsgemäßen Zusammensetzung, enthalten. Es ist erfindungsgemäß bevorzugt, wenn das anionische, filmbildende und/oder anionische, festigende Polymere mindestens eine Struktureinheit der Formel (S1) enthält, die ausgewählt wird aus mindestens einer Struktureinheit der Formeln (S1-1) bis (S1-5)

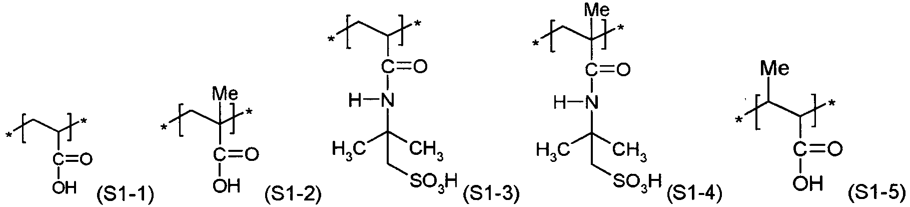

Es ist erfindungsgemäß bevorzugt, wenn das anionische, filmbildende und/oder anionische, festigende Polymere neben mindestens einer Struktureinheit der Formeln (S1-1) bis (S1-5) zusätzlich mindestens eine Struktureinheit der Formel (S2) enthält, die ausgewählt wird aus mindestens einer Struktureinheit der Formeln (S2-1) bis (S2-8) 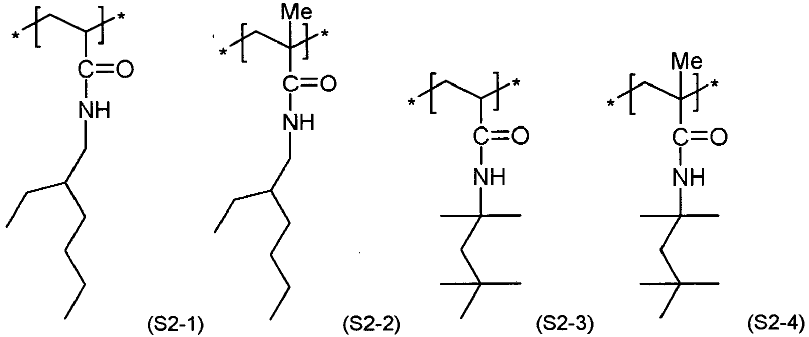 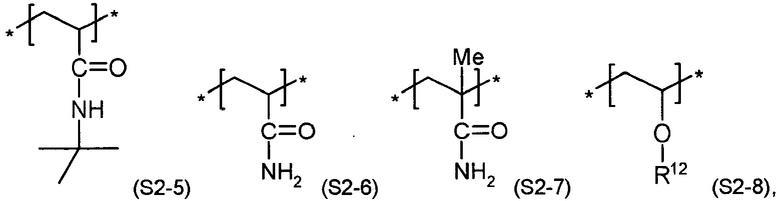 worin R¹² für eine (C₂ bis C₁₂)-Acylgruppe (insbesondere für Acetyl oder Neodecanoyl) steht.

Im Rahmen einer bevorzugten Ausführungsform gelten solche erfindungsgemäßen pulverförmigen Zusammensetzungen als erfindungsgemäß bevorzugt, die als in Form von Partikeln vorliegendes filmbildendes und/oder festigendes Polymer mindestens ein Polymer enthalten, das mindestens eine Struktureinheit der Formel (S1-5) und mindestens eine Struktureinheit der Formel (S2-8) enthalten 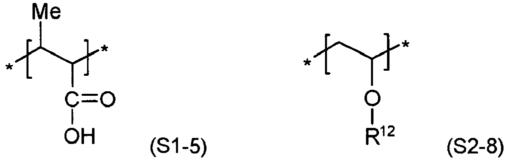 worin R¹² für eine (C₂ bis C₁₂)-Acylgruppe (insbesondere für Acetyl und/oder Neodecanoyl) steht. Besonders bevorzugte Polymere dieser Art werden ausgewählt aus mindestens einem Polymer der Gruppe, die gebildet wird aus
- Copolymeren aus Vinylacetat und Crotonsäure.
- Copolymeren aus Vinylpropionat und Crotonsäure,
- Copolymeren aus Vinylneodecanoat, Vinylacetat und Crotonsäure.

Solche Copolymere werden beispielsweise von der Firma Clariant unter dem Handelsnamen Aristoflex A 60 (INCI-Bezeichnung: VA/Crotonates Copolymer) in einem Isopropanol-Wasser-Gemisch (60 Gew.-% Aktivsubstanz), von der Firma BASF unter dem Handelsnamen Luviset CA 66 (Vinylacetat/Crotonsäure-Copolymer 90:10, INCI-Bezeichnung VA/Crotonates Copolymer) bereitgestellt, von der Firma National Starch unter dem Handelsnamen Resyn 28-2942 bzw. Resyn 28-2930 (INCI-Bezeichnung: VA/Crotonates/Vinyl Neodecanoate Copolymer) bereitgestellt.

Im Rahmen einer bevorzugten Ausführungsform gelten solche erfindungsgemäßen pulverförmigen Zusammensetzungen als erfindungsgemäß bevorzugt, die als anionisches filmbildendes und/oder anionisches festigendes Polymer mindestens ein Polymer enthalten, das mindestens eine Struktureinheit der Formel (S1-1) und mindestes eine Struktureinheit der Formel (S2-5) enthalten

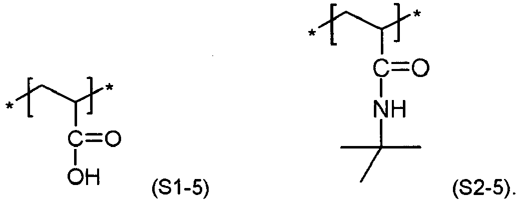

Dabei ist es wiederum besonders bevorzugt, wenn das in Form von Partikeln vorliegende, filmbildende und/oder festigende Polymer neben den obigen Struktureinheiten der Formeln (S1-1) und (S2-5) zusätzlich mindestens eine Struktureinheit der Formel (S3) enthält 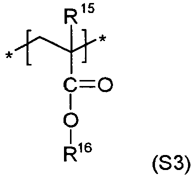 worin
R¹⁵ steht für ein Wasserstoffatom oder eine Methylgruppe
R¹⁶ steht für eine (C₁ bis C₄)-Alkylgruppe (insbesondere eine Methylgruppe oder eine Ethylgruppe).

Besonders bevorzugte Polymere dieser Art werden ausgewählt aus mindestens einem Polymer der Gruppe, die gebildet wird aus Copolymeren aus Acrylsäure und Ethylacrylat und N-tert.-Butylacrylamid. Solche Copolymere werden beispielsweise von der Firma BASF unter dem Handelsnamen Ultrahold^{®} Strong (INCI-Bezeichnung: Acrylates/t-Butylacrylamide Copolymer, weißes, schüttfähiges Granulat) oder Ultrahold^{®} 8 (INCI-Bezeichnung: Acrylates/t-Butylacrylamide Copolymer, weißes, schüttfähiges Granulat) bereitgestellt.

Im Rahmen einer Ausführungsform gelten solche Mittel als erfindungsgemäß bevorzugt, die als anionisches filmbildendes und/oder anionisches festigendes Polymer mindestens ein Polymer enthalten, das mindestens eine Struktureinheit der Formel (S1-3) und mindestens eine Struktureinheit der Formel (S2-6) enthalten

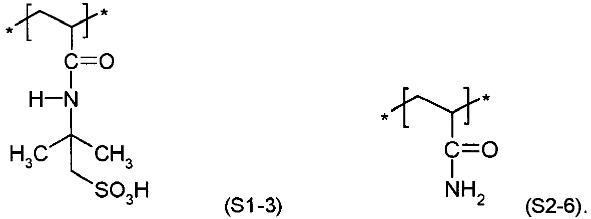

Bevorzugte Polymere (b) dieser Art werden ausgewählt aus mindestens einem Polymer der Gruppe, die gebildet wird aus
- Copolymeren aus 2-Acrylamido-2-methyl-propansulfonsäure und Acrylamid,
- Copolymeren aus 2-Acrylamido-2-methyl-propansulfonsäure, Acrylamid und Acrylsäure,
- Copolymeren aus 2-Acrylamido-2-methyl-propansulfonsäure, Acrylamid und Methacrylsäure. Polymere dieser Art werden beispielsweise in einer inversen Isohexadecan-Emulsion von der Firma Seppic unter dem Handelsnamen Sepigel^{®} 305 (INCI-Bezeichnung: Polyacrylamide, C13-14 Isoparaffin, Laureth-7) oder Simulgel^{®} 600 (INCI-Bezeichnung: Acrylamide/Acryloyldimethyltaurate Copolymer, Isohexadecane, Polysorbate-80) vertrieben.

Ein erfindungsgemäß besonders bevorzugtes Mittel ist dadurch gekennzeichnet, daß es als Polymer ein Copolymer (b5) enthält.

Diese Copolymere (b5) lassen sich durch die allgemeine Formel 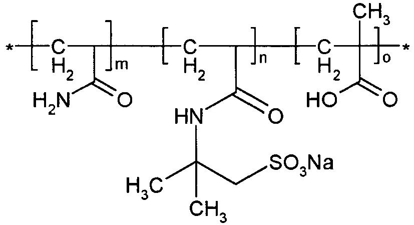 beschreiben, wobei die Indices m, n und o je nach Molmasse des Polymers variieren und nicht bedeuten sollen, daß es sich um Blockcopolymere handelt. Vielmehr können die Struktureinheiten im Molekül statistisch verteilt vorliegen.

Besonders bevorzugte erfindungsgemäße Mittel sind dadurch gekennzeichnet, daß das Copolymer (b1) eine Molmasse von 50 bis 500 kDa, vorzugsweise von 100 bis 450 kDa, weiter bevorzugt von 150 bis 400 kDa und insbesondere von 200 bis 300 kDa aufweist.

Copolymere von Acrylamid mit Methacrylsäure und Acryloyldimethyltaurat sind beispielsweise unter dem Handelsnamen Acudyne® SCP (Rohm & Haas) erhältlich.

Im Rahmen einer weiteren Ausführungsform enthalten die erfindungsgemäßen pulverförmigen Zusammensetzungen als filmbildendes und/oder festigendes Polymer mindestens ein filmbildendes amphoteres und/oder festigendes amphoteres Polymer.

Unter einem amphoteren Polymer wird erfindungsgemäß ein Polymer verstanden, das in einem protischen Lösemittel bei Standardbedingungen Struktureinheiten mit anionischen Gruppen, welche durch Gegenionen unter Erhaltung der Elektroneutralität kompensiert werden müssen, trägt und zusätzlich Struktureinheiten mit durch Protonierung kationisierbaren Gruppen aufweist, jedoch frei von permanent kationisierten Gruppen ist. Unter anionische Gruppen fallen Carboxyl- und Sulfonsäuregruppen. Unter permanent kationisierten Stickstoffatomen sind solche Stickstoffatome zu verstehen, die eine positive Ladung tragen und dadurch eine quartäre Ammoniumverbindung bilden.

Die amphoteren, filmbildenden und/oder amphoteren, festigenden Polymere sind in der erfindungsgemäßen pulverförmigen Zusammensetzung bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht der erfindungsgemäßen Zusammensetzung, enthalten.

Es ist erfindungsgemäß geeignet, wenn das amphotere, filmbildende und/oder amphotere, festigende Polymer mindestens eine Struktureinheit der Formel (S1) enthält, die ausgewählt wird aus mindestens einer Struktureinheit der Formeln (S1-1) bis (S1-5)

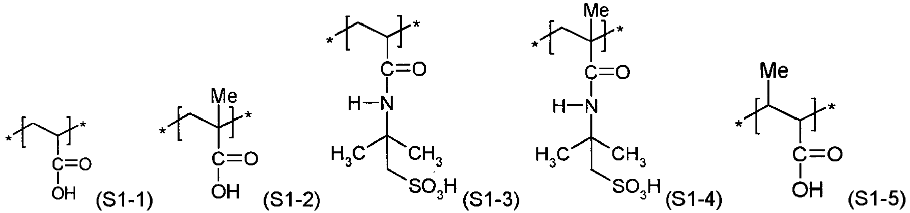

Es ist erfindungsgemäß geeignet, wenn das amphotere, filmbildende und/oder amphotere, festigende Polymere neben mindestens einer Struktureinheit der Formeln (S1-1) bis (S1-5) zusätzlich mindestens eine Struktureinheit der Formel (S2) enthält, die ausgewählt wird aus mindestens einer Struktureinheit der Formel (S2-9) bis (S2-15) 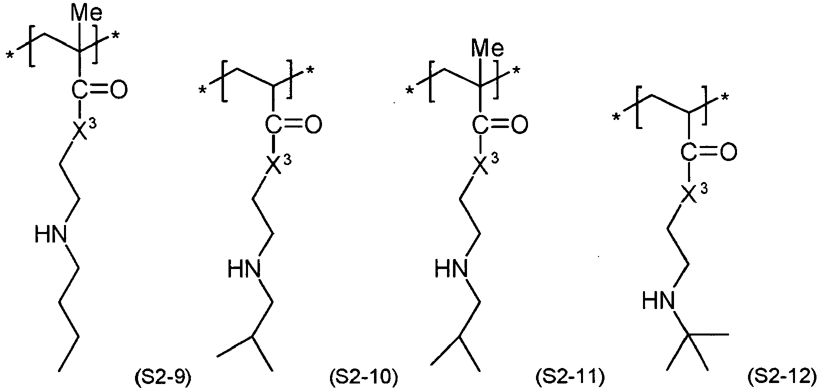 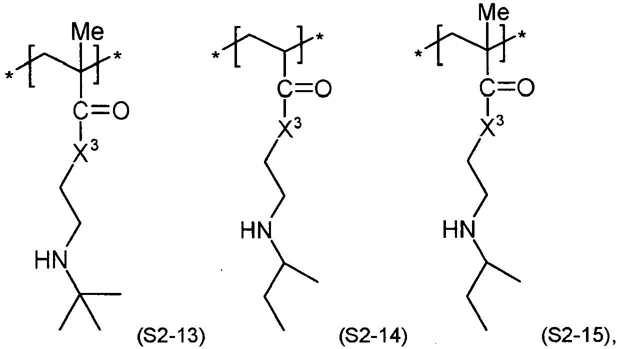 worin X³ steht für ein Sauerstoffatom oder eine Gruppe NH. Es ist wiederum erfindungsgemäß geeignet, wenn das amphotere, filmbildende und/oder amphotere, festigende Polymere neben mindestens einer Struktureinheit der Formeln (S1-1) bis (S1-5) und mindestens einer Struktureinheit der Formeln (S2-9) bis (S2-15) zusätzlich mindestens eine Struktureinheit der Formeln (S2-1) bis (S2-8) umfasst 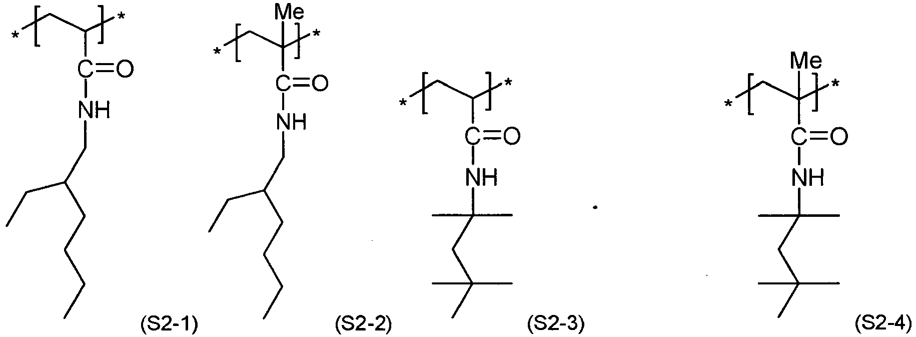 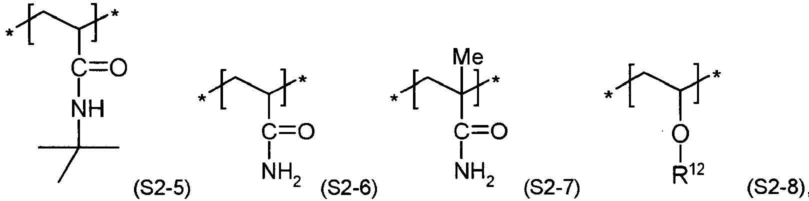 worin R¹² für eine (C₂ bis C₁₂)-Acylgruppe (insbesondere für Acetyl oder Neodecanoyl) steht.

Im Rahmen einer weiteren Ausführungsform der Erfindung enthält die erfindungsgemäße pulverförmige Zusammensetzung mindestens ein amphoteres, filmbildendes und/oder amphoteres, festigendes Polymer, welches mindestens eine Struktureinheit der Formel (S1-1), mindestens eine Struktureinheit der Formel (S2-3) und mindestens eine Struktureinheit der Formel (S2-16) (insbesondere ausgewählt aus der Gruppe, die gebildet wird aus obigen Formeln (S2-5) bis (S2-12) mit der Maßgabe, dass X³ für ein Sauerstoffatom steht), 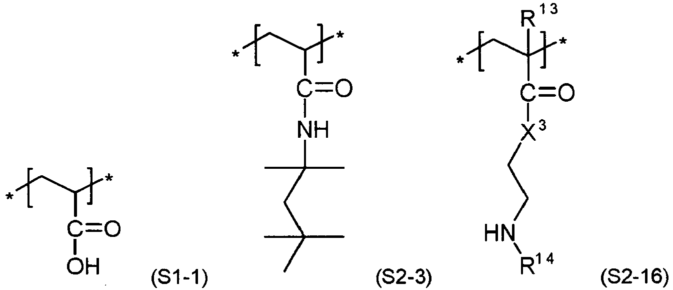 worin X³ steht für ein Sauerstoffatom oder eine Gruppe NH,
R¹³ steht für ein Wasserstoffatom oder eine Methylgruppe und
R¹⁴ steht für eine Alkylgruppe mit 4 Kohlenstoffatomen (insbesondere n-Butyl, sec-Butyl, iso-Butyl oder tert-Butyl).

Dabei ist es wiederum besonders geeignet, wenn das amphotere, filmbildende und/oder anionische, festigende Polymere neben den obigen Struktureinheiten der Formeln (S1-1), (S2-3) und (S2-16) zusätzlich mindestens eine Struktureinheit der Formel (S3) enthält 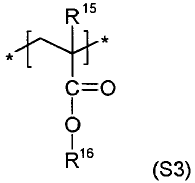 worin
R¹⁵ steht für ein Wasserstoffatom oder eine Methylgruppe
R¹⁶ steht für eine (C₁ bis C₄)-Alkylgruppe (insbesondere eine Methylgruppe oder eine Ethylgruppe).

Bevorzugte Polymere dieser Art werden ausgewählt aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.

Ein Beispiel eines besonders bevorzugt im Rahmen dieser Ausführungsform verwendbaren in Form von Partikeln vorliegenden, filmbildenden und/oder festigenden Polymers ist das unter dem Handelsnamen Amphomer^{®} von der Firma National Starch erhältliche Polymer mit der INCI-Bezeichnung Octylacrylamide/Acrylates/Butylaminoethylmethacrylate Copolymer.

Pulverförmige Zusammensetzungen, die zusätzlich mindestens ein Monosaccharid und/oder mindestens ein Disaccharid enthalten, bewirken insbesondere verbesserte Eigenschaften der resultierenden Frisur.

Es können sowohl Monosaccharide als auch Disaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Die Mono- bzw. Disaccharide sind in den erfindungsgemäßen pulverförmigen Zusammensetzungen bevorzugt in einer Menge von 0,1 bis 8 Gew.-%, insbesondere 1 bis 5 Gew.-%, bezogen auf die erfindungsgemäße pulverförmige Zusammensetzung, enthalten. Ähnliche Effekte können auch durch Zusatz mindestens eines Zuckeralkohols abgeleitet von Pentosen und/oder von Hexosen, erzielt werden. Bevorzugte Zuckeralkohole sind Sorbitol, Mannit und Dulcit, besonders bevorzugt einsetzbar ist Sorbitol.

Die Zuckeralkohole abgeleitet von Pentosen und/oder von Hexosen sind in den erfindungsgemäßen pulverförmigen Zusammensetzungen bevorzugt in einer Menge von 0,1 bis 8 Gew.-%, insbesondere 1 bis 5 Gew.-%, bezogen auf die erfindungsgemäße pulverförmige Zusammensetzung, enthalten.

Erfindungsgemäß bevorzugte pulverförmige Zusammensetzungen enthalten zusätzlich mindestens ein Alkalisierungsmittel.

Die erfindungsgemäß verwendbaren Alkalisierungsmittel und werden bevorzugt ausgewählt aus der Gruppe, die gebildet wird, aus basischen Aminosäuren, Alkalihydroxiden, Alkanolaminen, Alkalimetallmetasilikaten, Harnstoff, Morpholin, N-Methylglucamin, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Triethanolamin, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe Triethanolamin, 2-Aminoethan-1-ol, 2-Amino-2-methylpropan-l-ol und 2-Amino-2-methyl-propan-1,3-diol.

Besonders bevorzugt wird das Alkalisierungsmittel ausgewählt aus mindestens einer Verbindung aus der Gruppe, die gebildet wird aus Triethanolamin, 2-Aminoethanol, 2-Amino-2-methylpropan-1-ol, 2-Amino-2-methyl-propan-1,3-diol, Kaliumhydroxid, L-Arginin, D-Arginin, DL-Arginin.

Ferner ist es erfindungsgemäß bevorzugt, wenn die erfindungsgemäße pulverförmige Zusammensetzung zusätzlich mindestens ein Extrakt aus Algen enthält. Dieses Algenextrakt wird wiederum bevorzugt durch Extraktion von roten Algen und/oder von braunen Algen gewonnen. Ganz besonders bevorzugt ist Algenextrakt mit der CAS-Nummer 92128-82-0. Dieses wir beispielsweise in dem Handelsprodukt Fucosorb^{®} der Firma Sensient / LCW vertrieben.

Die erfindungsgemäßen pulverförmigen Zusammensetzungen enthalten bevorzugt 0,1 bis 4 Gew.-% Algenextrakt bezogen auf das Gewicht der pulverförmigen Zusammensetzung.

Die auf den Seiten 34 bis 48 der Offenlegungsschrift genannten Ausführungsformen (A) bis (DD) stellen ganz besonders bevorzugte Ausführungsformen der erfindungsgemäßen pulverförmigen Zusammensetzung dar.

Es ist erfindungsgemäß bevorzugt, wenn jede der Ausführungsformen (A) bis (Z) bzw. (AA) bis (DD) zusätzlich mindestens ein Algenextrakt und zusätzlich Sorbitol enthält.

Es ist erfindungsgemäß bevorzugt, wenn jede der Ausführungsformen (A) bis (Z) bzw. (AA) bis (DD) zusätzlich mindestens ein Alkalisierungsmittel, insbesondere Triethanolamin, enthält.

Es ist erfindungsgemäß bevorzugt, wenn jede der Ausführungsformen (A) bis (Z) bzw. (AA) bis (DD) zusätzlich mindestens ein Algenextrakt und zusätzlich Sorbitol und zusätzlich mindestens ein Alkalisierungsmittel, insbesondere Triethanolamin, enthält.

Die erfindungsgemäße pulverförmige Zusammensetzung kann generell weiterhin die Hilfs- und Zusatzstoffe enthalten, die üblicherweise herkömmlichen Stylingmitteln zugesetzt werden.

Dazu gehören kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Bevorzugt sind die kationischen Proteinhydrolysate und -derivate auf pflanzlicher Basis.

Als Pflegestoff kann weiterhin mindestens ein Vitamin, ein Provitamin, eine Vitaminvorstufe und/oder eines derer Derivate eingesetzt werden.

Dabei sind erfindungsgemäß solche Vitamine, Pro-Vitamine und Vitaminvorstufen bevorzugt, die üblicherweise den Gruppen A, B, C, E, F und H zugeordnet werden. Besonders bevorzugt sind Vitamine, die zur B-Gruppe oder zu dem Vitamin B-Komplex gehören, ganz besonders bevorzugt Vitamin B₅ (Pantothensäure, Panthenol und Pantolacton).

Als Pflegestoff kann weiterhin mindestens ein Pflanzenextrakt eingesetzt werden.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Seerose, Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Weitere geeignete Pflegestoffe sind Proteinhydrolysate und/oder deren Derivate, wobei die Verwendung von Proteinhydrolysaten pflanzlichen Ursprungs, z. B. Soja-, Mandel-, Erbsen-, Kartoffel- und Weizenproteinhydrolysaten, bevorzugt ist. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Neben den Pflegestoffen können auch weitere Hilfs- und Zusatzstoffe zugegeben werden.

Durch Zugabe eines UV-Filters können sowohl die Zubereitungen selbst, als auch die behandelten Fasern vor schädlichen Einflüssen von UV-Strahlung geschützt werden. Es kann daher vorteilhaft sein, den pulverförmigen Zubereitungen mindestens einen UV-Filter zuzugeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestem, Diphenylacrylsäureestern, Zimtsäureestern, Salicylsäureestern, Benzimidazolen und o-Aminobenzoesäureestern. Beispielhaft sei hier 2-Hydroxy-4-methoxybenzophenon-5-sulfonsäure und deren Natriumsalz (Benzophenone-4; Uvinul^{®}MS 40; Uvasorb^{®}S 5) genannt.

In einer besonderen Ausführungsform enthält die eingesetzte pulverförmige Zusammensetzung weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Die pulverförmigen Zusammensetzungen, die erfindungsgemäß zur temporären Verformung keratinischer Fasern eingesetzt werden, lassen sich einfach herstellen. Es hat sich bei Einsatz unterschiedlicher erfindungsgemäß beschichteter Metalloxidpartikel bewährt, zunächst alle erfindungsgemäß beschichteten Metalloxidpartikel zu vermischen. Dann werden in einem separaten Mischer alle Inhaltsstoffe bis auf besagte erfindungsgemäß beschichtete Metalloxidpartikel in die wässrige Phase unter Rühren eingearbeitet. Schließlich werden die beschichteten Metalloxidpartikel unter intensivem Rühren zu der wässrigen Phase gegeben. Die benötigte Mischzeit ist abhängig von der eingebrachten Mischenergie und der jeweiligen Zusammensetzung der Mischung, beträgt aber in der Regel zwischen 15 Sekunden und 5 Minuten. Wird zu kurz gemischt, bildet sich kein stabiles Pulver aus und es kommt zur Ausbildung einer öligen Phase. Bei zu langer Mischzeit wird das zunächst entstehende Pulver in eine brei- oder cremeartige Konsistenz überführt, wobei dieser Vorgang nicht reversibel verläuft. Es empfiehlt sich daher, durch einige Vorversuche die optimale Mischzeit für das jeweilige System zu ermitteln.

Die pulverförmigen Zusammensetzungen können in nahezu beliebigen Behältnissen konfektioniert werden. Es muss lediglich sichergestellt werden, dass die mechanische Belastung des Pulvers bei der Entnahme der Zusammensetzung nicht so hoch ist, dass bereits bei der Entnahme das Pulver in flüssige Form überführt wird. Geeignet sind beispielsweise Tiegel, Flaschen oder auch Tetrapacks, wobei das Behältnis beispielsweise mit einer Schütt- und Dosiervorrichtung ausgestaltet werden kann. Bei der Verwendung der pulverförmigen Zusammensetzung zur temporären Verformung des Haupthaars wird zunächst die gewünschte Menge der pulverförmigen Zusammensetzung dem Behältnis entnommen. Die Zusammensetzung kann dabei direkt auf das zu behandelnde Haupthaar oder aber beispielsweise auf die Hand gegeben werden. Im ersten Fall kann das aufgebrachte Pulver direkt auf dem Haupthaar einer mechanischen Belastung, beispielsweise mittels der Hände ausgesetzt werden, wodurch die wässrige Phase direkt auf der Haarfaser freigesetzt wird. Wird die pulverförmige Zusammensetzung zunächst auf die Hand gegeben, so kann es zunächst vorsichtig im Haar verteilt und wiederum erst anschließend stärker mechanisch belastet werden, beispielsweise durch gezieltes Einmassieren des Pulvers in das Haar. So lässt sich sehr gezielt eine hervorragende Stylingwirkung erreichen. Es ist natürlich auch möglich, die pulverförmige Zusammensetzung bereits auf der Hand zu verreiben und erst das entstehende flüssige oder pastenartige Mittel auf das Haupthaar aufzubringen. Diese Vorgehensweise ist allerdings nicht bevorzugt, da dabei auf einen wesentlichen Vorteil der pulverförmigen Konsistenz des Stylingmittels, nämlich die gute Verteilbarkeit, verzichtet wird. Die pulverförmige Zusammensetzung lässt sich natürlich auch mit einem Hilfsmittel, etwa einem Pinsel, einem Schwamm, einem Tuch, einer Bürste oder einem Kamm auftragen.

Ein zweiter Gegenstand ist die Verwendung einer pulverförmigen Zusammensetzung des ersten Erfindungsgegenstandes zur Erzeugung von Glanz auf menschlichem Haupthaar.

Ein dritter Gegenstand ist die Verwendung einer pulverförmigen Zusammensetzung des ersten Erfindungsgegenstandes zur temporären Umformung von menschlichem Haupthaar.

Ein vierter Gegenstand ist ein Verfahren zur temporären Umformung und/oder Glanzgebung menschlichen Haupthaars, dadurch gekennzeichnet, dass eine pulverförmige Zusammensetzung des ersten Erfindungsgegenstandes vor oder während der Applikation auf das Haupthaar mechanisch, insbesondere durch Druck oder Reibung, unter Freisetzung einer flüssigen Zusammensetzung beansprucht wird und die aus der mechanischen Beanspruchung der pulverförmigen Zusammensetzung entstandene flüssige Zusammensetzung auf das Haupthaar einwirkt.

Dabei ist es erfindungsgemäß bevorzugt, dass das Haupthaar nach der Applikation nicht gespült wird.

Die nachfolgenden Beispiele sollen den Gegenstand der vorliegenden Erfindung erläutern ohne ihn in irgendeiner Weise zu beschränken.

### Beispiele

Folgende Formulierung wurde als glanzbringendes Haarstylingpulver durch mischen bereitgestellt. Dabei wurden zunächst die pulverförmigen Rohstoffe PW F-MS^{®}, Covapearl Bright 933 AS^{®} und Mica 8 AS R0433^{®} vermischt. In einem separaten Behältnis wurden die restlichen Inhaltsstoffe miteinander vermischt. Zu dieser flüssigen Mischung wurde unter Rühren (Propellerrührer, 650 rpm) die Mischung der pulverförmigen Rohstoffe zugegeben. Es wurde solange Rühren gelassen (ca. 10 Minuten), bis eine fließfähige pulverförmige Zusammensetzung entstand.

| | Gew.-% |
|---|---|
| PW F-MS^{® 1} | 3,00 |
| Covapearl Bright 933 AS^{® 2} | 2,00 |
| Mica 8 AS R0433^{® 3} | 9,50 |
| Covagel^{® 4} | 2,00 |
| Fucosorb^{® 5} | 2,00 |
| Glyzerin | 5,00 |
| Triethanolamin | 0,07 |
| Neolone PE^{® 6} | 0,30 |
| Methylparaben | 0,10 |
| Wasser | 76,03 |

| | |
|---|---|
| ¹ mit Polyperfluoromethylisopropylether (CAS-Nr.: 69991-67-9, EINECS 274-225-4) und Triethoxycaprylylsilan beschichtetes, nanofeines Titandioxid (INCI-Bezeichnung: CI 77891, Polyperfluoromethylisopropyl ether, Triethoxycaprylylsilane) (Sensient / LCW) ² mit Triethoxycaprylylsilan beschichtete mineralische Perlglanzpigmente aus 25-35% Titandioxid, 65-75% Mica, 2% Triethoxycäprylylsilan (INCI-Bezeichnung: CI 77891, Mica, Triethoxycaprylylsilane) (Sensient / LCW) ³ mit Triethoxycaprylylsilan beschichteter Glimmer aus 99% Mica, 1 % Triethoxycaprylylsilan (INCI-Bezeichnung: Mica, Triethoxycaprylylsilan) (Sensient / LCW) ⁴ Carboxymethylstärke, Natriumsalz, als Stärkequelle diente Kartoffelstärke (INCI-Bezeichnung: Sodium Carboxymethyl Starch) (Sensient /LCW) ⁵ aus roter und brauner Alge gewonnener Algenextrakt kombiniert mit Sorbitol, enthält biopolymere Elektrolyte und Polysaccharide (INCI-Bezeichnung: Algae, Sorbitol) (Sensient/LCW) ⁶ Gemisch aus 2-Phenoxyethanol und Methylisothiazolinone (enthält 81-86 Gew.-% 2-Phenoxyethanol; INCI-Bezeichnung: Phenoxyethanol, Methylisothiazolinone) (Rohm & Haas) | |

Das erhaltene Haarstylingpulver wurde in einen Behälter aus Polyethylen gefüllt. Aus dem Behälter wurde das Pulver auf Haarpartien eines Probanden lokal aufgetragen. Das Pulver haftete am Haar. Die mit dem Pulver eingepuderten Haarpartien wurden mit der Hand geknetet und das wässrige Mittel im Kern der Pulverpartikel selektiv auf den besagten Haarpartien freigesetzt. Das Haar wurde während des Knetens in die gewünschte Form gebracht. Es wurde eine Frisur erhalten, die durch das Haarstylingpulver in ihrer Form stabilisiert war. Das Haar wies einen Glanzeffekt auf.

## Patentansprüche

1. Pulverförmige Zusammensetzung zur temporären Verformung des menschlichen Haupthaars, enthaltend
i) 50 bis 90 Gew.-% Wasser,
ii) 0,5 Gew.-% bis 10 Gew.% mindestens einer weiteren Flüssigkeit, die einen Siedepunkt von mehr als 150°C bei einem Druck von 1013 mbar aufweist,
iii) mindestens eine Stärke, die mit Carboxy-(C₁ bis C₄)-alkylgruppen substituiert ist,
iv) Metalloxidpartikel, die an der Oberfläche mit fluorhaltigen, organischen Gruppen beschichtet wurden,
v) mittels Triethoxycaprylsilan beschichtete Metalloxidpartikel, die keine fluorierten organischen Gruppen tragen.

2. Pulverförmige Zusammensetzung nach Anspruch 1, **dadurch gekennzeichnet, dass** die weitere Flüssigkeit mit einem Siedepunkt von mehr als 150°C bei einem Druck von 1013 mbar aus mindestens einem (C₃ bis C₈)-Alkohol mit mindestens 2 Hydroxylgruppen ausgewählt wird.

3. Pulverförmige Zusammensetzung nach einem der Ansprüche 1 oder 2, **dadurch gekennzeichnet, dass** die weitere Flüssigkeit mit einem Siedepunkt von mehr als 150°C bei einem Druck von 1013 mbar Glyzerin ist.

4. Pulverförmige Zusammensetzung nach einem der Ansprüche 1 bis 3, **dadurch gekennzeichnet, dass** die mit Carboxy-(C₁ bis C₄)-alkylgruppen substituierte Stärke ausgewählt wird aus mindestens einer Stärke aus der Gruppe Carboxymethylstärke, Carboxyethylstärke, Carboxypropylstärke.

5. Pulverförmige Zusammensetzung nach einem der Ansprüche 1 bis 4, **dadurch gekennzeichnet, dass** die Metalloxidpartikel an der Oberfläche durch Umsetzung mit mindestens einem Reagenz der Formel (I) beschichtet wurden 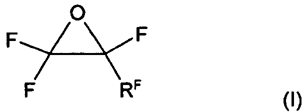 worin
R^{F} für eine Perfluor-(C₁ bis C₄)-alkylgruppe, insbesondere Trifluormethyl, steht.

6. Pulverförmige Zusammensetzung nach einem der Ansprüche 1 bis 4, **dadurch gekennzeichnet, dass** die Metalloxidpartikel an der Oberfläche durch mindestens eine Verbindung der Formel (Ia) beschichtet sind 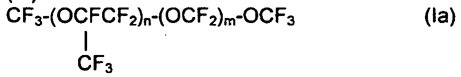 worin die Summe n + m für eine ganze Zahl von 20 bis 80 steht.

7. Pulverförmige Zusammensetzung nach einem der Ansprüche 1 bis 6, **dadurch gekennzeichnet, dass** es zusätzlich Metalloxidpartikel enthält, die durch Umsetzung mit mindestens einem Reagens der Formel (II) beschichtet wurden
(R¹O)ₙ(CH₃)₃₋ₙSi-R² (II)
worin
R¹ für Methyl oder Ethyl steht,
R² für eine (C₁ bis C₁₄)-Alkylgruppe, insbesondere eine (C₆ bis C₁₂)-Alkylgruppe, steht,
n für 1, 2 oder 3 steht.

8. Pulverförmige Zusammensetzung nach Anspruch 7, **dadurch gekennzeichnet, dass** die Metalloxidpartikel an der Oberfläche durch Umsetzung mit mindestens einem Reagenz der Formel (Ia) und mindestens einem Reagenz der Formel (II) beschichtet wurden.

9. Pulverförmige Zusammensetzung nach einem der Ansprüche 1 bis 8, **dadurch gekennzeichnet, dass** besagte beschichtete Metalloxidpartikel einen Partikeldurchmesser von weniger als 5 µm, bevorzugt weniger als 1 µm, besonders bevorzugt zwischen 20 und 100 nm, aufweisen.

10. Pulverförmige Zusammensetzung nach einem der Ansprüche 1 bis 9, **dadurch gekennzeichnet, dass** die mit fluorhaltigen, organischen Gruppen beschichteten Metalloxidpartikel ausgewählt werden aus mindestens einem Vertreter der Gruppe, die gebildet wird aus mit fluorhaltigen, organischen Gruppen beschichteten Aluminaten, mit fluorhaltigen, organischen Gruppen beschichteten Silikaten, mit fluorhaltigen, organischen Gruppen beschichteten Aluminiumsilikaten, mit fluorhaltigen, organischen Gruppen beschichtetem Titandioxid sowie mit fluorhaltigen, organischen Gruppen beschichtetem Silikagel..

11. Pulverförmige Zusammensetzung nach einem der Ansprüche 1 bis 10, **dadurch gekennzeichnet, dass** besagte beschichtete Metalloxidpartikel insgesamt in einer Menge von 5 Gew.-% bis 25 Gew.-% enthalten sind.

12. Pulverförmige Zusammensetzung nach einem der Ansprüche 1 bis 11, **dadurch gekennzeichnet, dass** sie zusätzlich mindestens ein filmbildendes und/oder festigendes Polymer enthält.

13. Pulverförmige Zusammensetzung nach einem der Ansprüche 1 bis 12, **dadurch gekennzeichnet, dass** sie Kern-Hülle-Teilchen umfasst, deren Hülle besagte Metalloxidpartikel enthält und deren Kern eine flüssige, wässrige Phase umfasst.

14. Verfahren zur temporären Umformung und/oder Glanzgebung menschlichen Haupthaars, **dadurch gekennzeichnet, dass** eine pulverförmige Zusammensetzung nach einem der Ansprüche 1 bis 13 vor oder während der Applikation auf das Haupthaar mechanisch, insbesondere durch Druck oder Reibung, unter Freisetzung einer flüssigen Zusammensetzung beansprucht wird und die aus der mechanischen Beanspruchung der pulverförmigen Zusammensetzung entstandene flüssige Zusammensetzung auf das Haupthaar einwirkt.

15. Verfahren nach Anspruch 14, **dadurch gekennzeichnet, dass** das Haupthaar nach der Applikation nicht gespült wird.

## Claims

1. A powdery composition for temporarily shaping human hair, containing
i) 50 to 90 wt.% of water,
ii) 0.5 wt.% to 10 wt.% of at least one further liquid, which has a boiling point higher than 150°C at a pressure of 1013 mbar,
iii) at least one starch substituted with carboxy-(C₁ to C₄) alkyl groups,
iv) metal oxide particles, which have been coated on the surface with fluorine-containing organic groups.
v) metal oxide particles which have been coated by triethoxycaprylsilane which do not bear any organic groups.

2. The powdery composition according to claim 1, **characterized in that** the further liquid having a boiling point higher than 150°C at a pressure of 1013 mbar is selected from at least one (C₃ to C₈) alcohol having at least two hydroxyl groups.

3. The powdery composition according to one of claims 1 or 2, **characterized in that** the further liquid having a boiling point higher than 150°C at a pressure of 1013 mbar is glycerol.

4. The powdery composition according to one of claims 1 to 3, **characterized in that** the starch substituted with carboxy-(C₁ to C₄) alkyl groups is selected from at least one starch from the group comprising carboxymethyl starch, carboxyethyl starch, carboxypropyl starch.

5. The powdery composition according to one of claims 1 to 4, **characterized in that** the metal oxide particles have been coated on the surface by reaction with at least one reagent of formula (I) 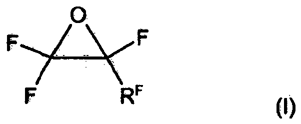 in which
R^{F} denotes a perfluoro-(C₁ to C₄) alkyl group, in particular trifluoromethyl.

6. The powdery composition according to one of claims 1 to 4, **characterized in that** the metal oxide particles are coated on the surface with at least one compound of formula (Ia) 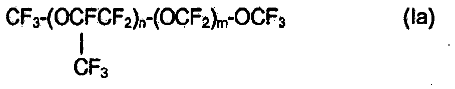 in which the sum n + m denotes a whole number from 20 to 80.

7. The powdery composition according to one of claims 1 to 6, **characterized in that** it additionally contains metal oxide particles which have been coated by reaction with at least one reagent of formula (II)
(R¹O)ₙ(CH₃)₃₋ₙSi-R² (II)
in which
R¹ denotes methyl or ethyl,
R² denotes a (C₁ to C₁₄) alkyl group, in particular a (C₆ to C₁₂) alkyl group,
n denotes 1, 2 or 3.

8. The powdery composition according to claim 7, **characterized in that** the metal oxide particles have been coated on the surface by reaction with at least one reagent of formula (Ia) and at least one reagent of formula (II).

9. The powdery composition according to one of claims 1 to 8, **characterized in that** said coated metal oxide particles have a particle diameter of less than 5 µm, preferably less than 1 µm, particularly preferably between 20 and 100 nm.

10. The powdery composition according to one of claims 1 to 9, **characterized in that** the metal oxide particles coated with fluorine-containing organic groups are selected from at least one representative of the group formed from aluminates coated with fluorine-containing organic groups, silicates coated with fluorine-containing organic groups, aluminum silicates coated with fluorine-containing organic groups, titanium dioxide coated with fluorine-containing organic groups, and silica gel coated with fluorine-containing organic groups.

11. The powdery composition according to one of claims 1 to 10, **characterized in that** said coated metal oxide particles are included in total in a portion from 5 wt.% to 25 wt.%.

12. The powdery composition according to one of claims 1 to 11, **characterized in that** it additionally contains at least one film-forming and/or fixing polymer.

13. The powdery composition according to one of claims 1 to 12, **characterized in that** it encompasses core-shell particles whose shell contains said metal oxide particles and whose core encompasses a liquid, aqueous phase.

14. A method for temporarily shaping and/or giving shine to human hair, **characterized in that** before or during application on the human hair a powdery composition according to one of claims 1 to 13 is subjected to mechanical loading, in particular by pressure or friction, with release of a liquid composition and the liquid composition arising from the mechanical loading of the powdery composition acts on the hair.

15. The method according to claim 14, **characterized in that** the hair is not rinsed following the application.

## Revendications

1. Composition sous forme de poudre pour la mise en forme temporaire des cheveux humains, contenant
i) 50 à 90 % en poids d'eau,
ii) 0,5 % en poids à 10 % en poids d'au moins un autre liquide, qui présente un point d'ébullition de plus de 150°C à une pression de 1013 mbars,
iii) au moins un amidon, substitué par des groupes carboxy-(C₁ à C₄)-alkyle,
iv) des particules d'oxyde de métal, qui ont été revêtues en surface par des groupes organiques, fluorés,
v) des particules d'oxyde de métal revêtues de triéthoxycaprylsilane, qui ne portent pas de groupes organiques, fluorés.

2. Composition sous forme de poudre selon la revendication 1, **caractérisée en ce que** l'autre liquide présentant un point d'ébullition de plus de 150°C à une pression de 1013 mbars est choisi parmi au moins un alcool en C₃-C₈ comprenant au moins 2 groupes hydroxyle.

3. Composition sous forme de poudre selon l'une quelconque des revendications 1 ou 2, **caractérisée en ce que** l'autre liquide présentant un point d'ébullition de plus de 150°C à une pression de 1013 mbars est le glycérol.

4. Composition sous forme de poudre selon l'une quelconque des revendications 1 à 3, **caractérisée en ce que** l'amidon substitué par des groupes carboxy-(C₁ à C₄)-alkyle est choisi parmi au moins un amidon du groupe carboxyméthylamidon, carboxyéthylamidon, carboxypropylamidon.

5. Composition sous forme de poudre selon l'une quelconque des revendications 1 à 4, **caractérisée en ce que** les particules d'oxyde de métal ont été revêtues en surface par transformation avec au moins un réactif de formule (I) 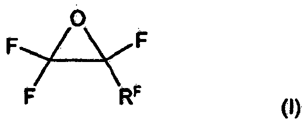 où
R^{F} représente un groupe perfluoro-(C₁ à C₄)-alkyle, en particulier trifluorométhyle.

6. Composition sous forme de poudre selon l'une quelconque des revendications 1 à 4, **caractérisée en ce que** les particules d'oxyde de métal sont revêtues en surface par au moins un composé de formule (la) 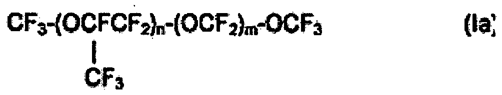 la somme n + m valant un nombre entier de 20 à 80.

7. Composition sous forme de poudre selon l'une quelconque des revendications 1 à 6, **caractérisée en ce qu'**elle contient en plus des particules d'oxyde de métal qui ont été revêtues par transformation avec au moins un réactif de formule (II)
(R¹O)ₙ(CH₃)₃₋ₙSi-R² (II)
où
R¹ représente méthyle ou éthyle,
R² représente un groupe (C₁ à C₁₄)-alkyle, en particulier un groupe (C₆ à C₁₂)-alkyle,
n vaut 1, 2 ou 3.

8. Composition sous forme de poudre selon la revendication 7, **caractérisée en ce que** les particules d'oxyde de métal ont été revêtues en surface par transformation avec au moins un réactif de formule (la) et au moins un réactif de formule (II).

9. Composition sous forme de poudre selon l'une quelconque des revendications 1 à 8, **caractérisée en ce que** lesdites particules d'oxyde de métal revêtues présentent un diamètre de particule inférieur à 5 µm, de préférence inférieur à 1 µm, de manière particulièrement préférée entre 20 et 100 nm.

10. Composition sous forme de poudre selon l'une quelconque des revendications 1 à 9, **caractérisée en ce que** les particules d'oxyde de métal revêtues de groupes organiques, fluorés sont choisies parmi au moins un représentant du groupe formé par les aluminates revêtus de groupes organiques fluorés, les silicates revêtus de groupes organiques fluorés, les aluminosilicates revêtus de groupes organiques fluorés, le dioxyde de titane revêtu de groupes organiques fluorés ainsi que le silicagel revêtu de groupes organiques fluorés.

11. Composition sous forme de poudre selon l'une quelconque des revendications 1 à 10, **caractérisée en ce que** lesdites particules d'oxyde de métal revêtues sont contenues au total en une quantité de 5 % en poids à 25 % en poids.

12. Composition sous forme de poudre selon l'une quelconque des revendications 1 à 11, **caractérisée en ce qu'**elle contient en outre au moins un polymère filmogène et/ou de renforcement.

13. Composition sous forme de poudre selon l'une quelconque des revendications 1 à 12, **caractérisée en ce qu'**elle contient des particules à noyau-enveloppe, dont l'enveloppe contient lesdites particules d'oxyde de métal et dont le noyau comprend une phase liquide, aqueuse.

14. Procédé pour la mise en forme temporaire et/ou pour donner de l'éclat aux cheveux humains, **caractérisé en ce qu'**une composition sous forme de poudre selon l'une quelconque des revendications 1 à 13 est sollicitée, avant ou pendant l'application sur les cheveux, mécaniquement, en particulier par pression ou frottement, avec libération d'une composition liquide et la composition liquide formée par la sollicitation mécanique de la composition sous forme de poudre agit sur les cheveux.

15. Procédé selon la revendication 14, **caractérisé en ce que** les cheveux ne sont pas rincés après l'application.
